(19) Europäisches Patentamt
European Patent Office
Office européen des brevets

(11) Veröffentlichungsnummer: **0 106 807 B1**

# (12) EUROPÄISCHE PATENTSCHRIFT

(45) Veröffentlichungstag der Patentschrift: **21.05.86**

(51) Int. Cl.⁴: **C 07 F 9/65**, A 01 N 57/16, A 01 N 57/24

(21) Anmeldenummer: **83810470.1**

(22) Anmeldetag: **10.10.83**

(54) **Neue Pflanzenschutzmittel, Verfahren zu ihrer Herstellung und ihre Verwendung.**

(30) Priorität: **15.10.82 CH 6021/82**

(43) Veröffentlichungstag der Anmeldung: **25.04.84 Patentblatt 84/17**

(45) Bekanntmachung des Hinweises auf die Patenterteilung: **21.05.86 Patentblatt 86/21**

(84) Benannte Vertragsstaaten: **BE CH DE FR IT LI NL**

(56) Entgegenhaltungen: **DE-A-3 046 329**

(73) Patentinhaber: **CIBA-GEIGY AG, Klybeckstrasse 141, CH-4002 Basel (CH)**

(72) Erfinder: **Eckhardt, Wolfgang, Dr., Breslauerstrasse 16, D-7850 Lörrach (DE)**
Erfinder: **Kunz, Walter, Dr., Im Goldbrunnen 55, CH-4104 Oberwil (CH)**

**EP 0 106 807 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Phosphorsäureester der nachstehenden Formel I sowie deren pflanzenverträgliche Säureadditionssalze, quartäre Azoliumsalze und Metallkomplexe. Sie betrifft ferner die Herstellung dieser Verbindungen sowie agrochemische Schädlingsbekämpfungsmittel, die als Wirkstoff mindestens eine der Verbindungen der Formel I enthalten; sie betrifft auch die Herstellung der Mittel und ein Verfahren zur Bekämpfung oder präventiven Verhütung eines Befalls von Pflanzen durch Schädlinge.

Bei den erfindungsgemässen Verbindungen handelt es sich um solche der Formel I

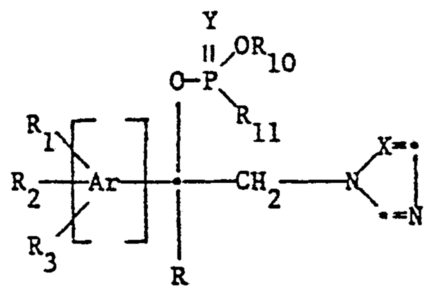

(I)

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen;

R eine der Gruppen $-COOR_4$, $-COSR_5$,

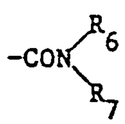

oder -CN darstellt;

$R_4$ ein unsubstituiertes oder durch Halogen substituiertes $C_2$-$C_{10}$-Alkenyl; eine unsubstituiertes oder durch Halogen substituiertes $C_2$-$C_{10}$-Alkinyl-, oder eine $C_3$-$C_8$-Cycloalkylgruppe oder eine unsubstituierte oder durch Halogen, $C_1$-$C_4$-Alkyl, $C_1$-$C_4$-Alkoxy, -CN oder $-CF_3$ substituierte Phenylgruppe bedeutet; oder aber eine $C_1$-$C_{12}$-Alkylkette darstellt, die ab $C_2$-Alkyl durch Sauerstoff oder Schwefel unterbrochen und die unsubstituiert oder durch eine der folgenden Atome oder Gruppen substituiert sein kann: Halogen, Phenyl, -COOAlkyl($C_1$-$C_4$), -COAlkyl($C_1$-$C_4$), -COPhenyl,einen ungesättigten oder gesättigten 5- oder 6-gliedrigen Ring mit Sauerstoff oder Schwefel als Heteroatom, wobei jeder Phenylrest unsubstituiert oder ein- oder mehrfach durch gleiche oder verschiedene Halogenatome substituiert ist;

$R_5$ $C_1$-$C_{10}$-Alkyl oder eine unsubstituierte oder durch Halogen, $C_1$-$C_4$-Alkyl, $C_1$-$C_4$-Alkoxy, -CN oder $-CF_3$ substituierte Phenyl- oder Benzylgruppe bedeutet;

$R_6$ und $R_7$ unabhängig voneinander Wasserstoff, $C_1$-$C_6$-Alkyl, $C_3$-$C_7$-Cycloalkyl oder eine Phenyl- oder Benzylgruppe darstellen, bei denen jeweils der aromatische Ring unsubstituiert oder durch Halogen, $C_1$-$C_4$-Alkyl, $C_1$-$C_4$-Alkoxy, -CN oder $-CF_3$ substituiert ist, oder wobei einer der Substituenten $R_6$ oder $R_7$ auch die Gruppe -$N(R_8)(R_9)$ bedeutet oder wobei die Substituenten $R_6$ und $R_7$ zusammen einen 5- oder 6-gliedrigen gesättigten oder ungesättigten heterocyclischen Ring bilden, der noch 1 oder 2 weitere N-Atome enthalten kann;

$R_8$ und $R_9$ unabhängig voneinander Wasserstoff, $C_1$-$C_4$-Alkyl odereinen Phenylrest bedeuten, der unsubstituiert oder durch Halogen, $C_1$-$C_4$-Alkyl, -CN oder $-CF_3$ substituiert ist;

$R_{10}$ $C_1$-$C_{12}$-Alkyl bedeutet;

$R_{11}$ für $-YR_{12}$, $C_1$-$C_{12}$-Alkyl oder Phenyl steht;

$R_{12}$ $C_1$-$C_{12}$-Alkyl bedeutet; und

Y für Sauerstoff oder Schwefel steht;

wobei pflanzenverträgliche Säureadditionssalze, quartäre Azolium- und Ammoniumsalze sowie Metallsalzkomplexe der Formel I mit eingeschlossen sind.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl, sowie ihre Isomeren, wie z.B. Isopropyl, Isobutyl, tert.-Butyl, Isopentyl Alkenyl steht z.B. für Vinyl, Propenyl-(1) Allyl Butenyl-(1), Butenyl-(2), Butenyl-(3) usw.. sowie Ketten mit mehreren Doppelbindungen. Alkinyl bedeutet z.B. Propinyl-(1), Propargyl, Buti-nyl-(1),

Butinyl-(2) usw.. bevorzugt Propargyl, durch Halogen substituiertes Alkyl steht insbesondere für einen ein- bis perhalogenierten Alkylsubstituenten, wie z.B. $CHCl_2$, $CH_2Cl$, $CCl_3$, $CF_3$, $CH_2CH_2Cl$, usw. Unter Halogen soll hier und im folgenden Fluor, Chlor, Brom oder Jod, vorzugsweise Cl, Br oder F verstanden werden. Cycloalkyl steht z.B. für Cyclopropyl, Cyclobutyl, Cyclopentyl, Cyclohexyl oder Cycloheptyl, bevorzugt für Cyclopropyl und Cyclohexyl. Haloalkenyl steht für eine ein- oder mehrfach durch Halogen substituierte Alkenylgruppe, wobei als Halogen Chlor und Brom, insbesondere Chlor bevorzugt sind. Furyl steht bevorzugt für 2-Furyl, Pyridyl bedeutet vor allem 3- oder 4-Pyridyl, Naphthyl steht für α- oder ß-Naphthyl, insbesondere für α-Naphthyl. Beispiele heterocyclischer 5- bzw. 6-Ringe mit bis zu 3 N-Atomen sind Pyrazol, Imidazol, 1,2,4-Triazol und 1,3,4-Triazol, Pyridin, Pyrazin, pyrimidin, Pyridazin, 1,3,5-Triazin und 1,2,4-Triazin.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glykolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsaure, 2-Phenoxybenzoesäure oder 2-Acetoxybenzoesäure.

Metallsalzkomplexe der Formel I bestehen aus dem zugrundeliegenden organischen Molekül und einem anorganischen oder organischen Metallsalz beispielsweise den Halogeniden, Nitraten, Sulfaten, Phosphaten, Acetaten, Trifluoracetaten, Trichlorscetaten, Propionaten, Tartraten, Sulfonaten, Salicylaten Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber. Bevorzugt sind die Nebengruppen-Elemente der 4. Periode. Die Metalle können dabei in den verschiedenen ihnen zukommenden Wertigkeiten vorliegen. Die Metallsalzkomplexe der Formel I können ein- oder mehrkernig auftreten, d.h. sie können ein oder mehrere organische Molekülanteile als Liganden enthalten. Komplexe mit den Metallen Kupfer, Zink, Mangan und Zinn sind bevorzugt.

Die Verbindungen der Formel I sind bei Raumtemperatur stabile Oele, Harze oder öberwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von Schädlingen, wie Pilzen, Insekten und Milben einsetzen, dabei sind die Triazolylmethylderivate im Umfang der Formel I (X bedeutet N) bevorzugt. Die erfindungsgemässen Wirkstoffe der Formel I zeichnen sich durch eine sehr gute Pflanzenverträglichkeit aus. Die Entwicklung der Pflanzen wird in keinem Stadium behindert oder verzögert.

Eine wichtige und bevorzugte Untergruppe betrifft Verbindungen der Formel I*

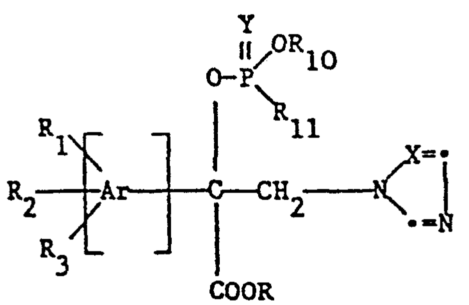

(I*) ,

worin

X für das Brückenglied -CH= oder -N= steht;
Ar eine Phenyl-, Diphenyl- oder Naphthylgruppe bedeutet;
$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen;
$R_4$ $C_1$-$C_4$-Alkyl, Phenyl, ein durch Nitro, Halogen und/ oder Methyl ein- oder mehrfach substituiertes Phenyl oder Benzyl bedeutet;
$R_{10}$ $C_1$-$C_6$-Alkyl bedeutet;
$R_{11}$ für -$YR_{12}$, $C_1$-$C_6$-Alkyl oder Phenyl steht;
$R_{12}$ $C_1$-$C_6$-Alkyl bedeutet; und
Y für Sauerstoff oder Schwefel steht;
unter Einschluss der pflanzenverträglichen Säureadditionsalze, quartäre Azolium- und Ammoniumsalze sowie Metallsalzkomplexe.

Eine andere bevorzugte Untergruppe bilden Verbindungen der Formel I, worin X für das Brückenglied -CH= oder -N= steht; Ar eine Phenylgruppe bedeutet; $R_1$ in ortho-Position für Wasserstoff, Nitro, Halogen, $C_1$-$C_3$-Alkyl, $C_1$-$C_3$-Alkoxy oder $C_1$-$C_3$-Haloalkyl steht; $R_2$ in para-Position für Wasserstoff, Nitro, Halogen, $C_1$-$C_3$-Alkyl, $C_1$-$C_3$-Alkoxy oder $C_1$-$C_3$-Haloalkyl steht; $R_3$ Wasserstoff, Methyl oder Halogen bedeutet; R eine der Gruppen -$COOR_4$, -$COSR_5$,

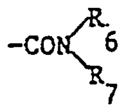

oder -CN darstellt; $R_4$ $C_1$-$C_4$-Alkyl, Phenyl, ein durch Nitro, Halogen und/oder Methyl ein- oder mehrfach substituiertes Phenyl oder Benzyl bedeutet; $R_5$ $C_1$-$C_{10}$-Alkyl oder eine unsubstituierte oder durch Halogen, $C_1$-$C_4$-Alkyl, $C_1$-$C_4$-Alkoxy, -CN oder -$CF_3$ substituierte Phenyl- oder Benzylgruppe bedeutet, R und R unabhängig voneinander für Wasserstoff, $C_1$-$C_3$-Alkyl, $C_3$-$C_7$-Cycloalkyl,Phenyl oder Benzyl stehen; $R_{10}$ C -$C_4$-Alkyl bedeutet; $R_{11}$ für -$YR_{12}C_1$-$C_4$-Alkyl oder Phenyl steht; $R_{12}$ $C_1$-$C_4$-Alkyl bedeutet; und Y für Sauerstoff oder Schwefel steht; wobei Säureadditionssalze, quartäre Azolium- und Ammoniumsalze sowie Metallkomplexe der Formel I mit eingeschlossen sind.

Eine weitere, besonders bevorzugte Untergruppe bilden Verbindungen der Formel I, worin X för das Brückenglied -N= steht;

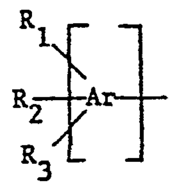

eine in ortho- und/oder para-Position durch Nitro, Fluor, Chlor, Brom, Methyl, Methoxy und/oder $CF_3$ substituierte Phenylgruppe bedeutet; R für die Gruppe -$COOR_4$ steht; $R_4$ $C_1$-$C_4$-Alkyl, Phenyl,ein durch Nitro, Chlor, Brom, Fluor und/oder Methyl substituiertes Phenyl oder ebenso substituiertes Benzyl bedeutet; $R_{10}$ $C_1$-$C_4$-Alkyl bedeutet; $R_{11}$ für -$YR_{12}$, $C_1$-$C_4$-Alkyl oder Phenyl steht; $R_{12}$ $C_1$-$C_4$-Alkyl bedeutet; und Y für Sauerstoff oder Schwefel steht; wobei Säureadditionssalze, quartäre Azolium- und Ammoniumsalze sowie Metallkomplexe der Formel I mit eingeschlossen sind.

Folgende Einzelsubstanzen sind als Mikrobizide besonders bevorzugt:

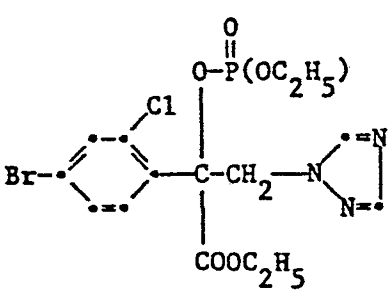 (1.3)

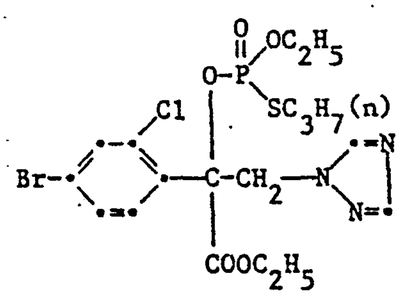 (1.19)

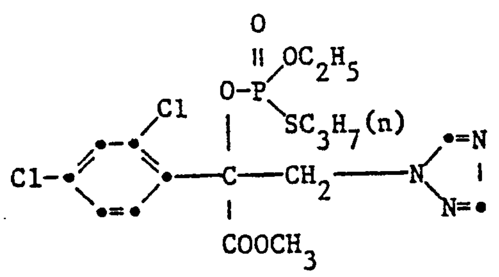 (1.51) 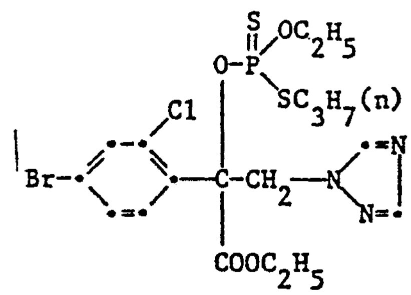 (1.22)

Die Verbindungen der Formel I werden erfindungsgemäss dadurch hergestellt, dass man einen Alkohol der Formel II

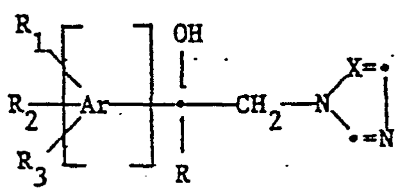 (II) ,

vorzugsweise in Gegenwart einer anorganischen oder insbesondere einer organischen Base bei Temperaturen von −20° bis +150°C, bevorzugt 0° bis 100°C, in Ab- oder vorzugsweise Anwesenheit eines reaktionsinerten organischen Lösungs- oder Verdünnungsmittel mit einem Phosphorsaurehalogenid der Formel III

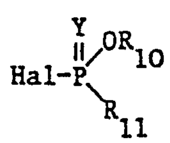 (III)

umsetzt und/oder eine verfahrensgemäss erhältliche freie Verbindung in ein pflanzenverträgliches Säureadditionssalz, quaternäres Azolium- oder Ammoniumsalz umwandelt, ein verfahrensgemäss erhältliches pflanzenverträgliches Säureadditionssalz, quaternäres Azolium- oder Ammoniumsalz, in die freie Verbindung oder ein anderes pflanzenverträgliches Säureadditionssalz, quaternäres Azolium- oder Ammoniumsalz, oder eine verfahrensgemäss erhältliche freie Verbindung bzw. ein verfahrensgemäss erhältliches Salz in einen Metallsalzkomplex überföhrt. Hierbei haben die Substituenten R, $R_1$, $R_2$, $R_3$, $R_4$, $R_{10}$, $R_{11}$, Y, X und Ar in den Formeln II und III die unter Formel I angegebenen Bedeutungen, und Hal in Formel III steht för ein Halogenatom, insbesondere für Chlor oder Brom.

Für die Umsetzung geeignete Lösungsmittel sind z.B. aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether), Anisol, Dioxan, Tetrahydrofuran; Nitrile wie Acetonitril, Propionitril; N,N-dialkyl-/ ierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid. Ferner kann das Arbeiten unter erhöhtem Druck sich gönstig auf die Ausbeute auswirken.

Geeignete anorganische Basen sind beispielsweise Oxide, Hydride, Hydroxide, Carbonate, Carbonsäuresalze und Alkoholate der Erdalkali-, bevorzugt der Alkalimetalle, insbesondere die des Natriums und Kaliums [z.B. NaH, NaOH, KOH, $Na_2CO_3$, $K_2CO_3$. $CaCO_3$, $CH_3COONa$, $C_2H_5COOK$,. $C_2H_5ONa$, $CH_3ONa$, ], bevorzugt die Alkalihydride wie NaH. Als organische Basen eignen sich Triälkylamine wie z.B. Triethylamin oder andere tertiäre Amine wie beispielsweise Triethylendiamin, Piperidin, Pyridin, 4-Dimethylaminopyridin, 4-Pyrrolidylpyridin.

Bei den erfindungsgemässen Herstellungsverfahren för die Endprodukte sowie bei der Herstellung der Ausgangsstufen können die anfallenden Zwischen- und End-Produkte aus dem Reaktionsmedium isoliert und,

falls gewünscht, auf eine der allgemein üblichen Methoden gereinigt werden, z.B. durch Extraktion, Kristallisation, Chromatographie, Destillation.

Besonders vorteilhafte Reaktionsvarianten zur Herstellung der Verbindungen der Formel I sowie zur Herstellung der Zwischenprodukte, insbesondere derjenigen der Formel II, werden anschliessend in zwei Reaktionsschemata skizziert und darauffolgend im einzelnen beschrieben.

In den Formeln Ia bis Ih, II, III,IV, V, VI, VII, VIII, IX, X, XI, XII, XIII und XIV haben die Substituenten Ar, X, R, $R_1$, $R_2$, $R_3$, $R_4$, $R_5$, $R_6$, $R_7$, $R_8$, $R_{10}$, $R_{11}$ und Y die unter Formel I angegebene Bedeutungen.

Q steht in Formel XV entweder für eine der üblichen Abgangsgruppen, z.B. Halogen, insbesondere Chlor, Brom oder Jod; für eine Sulfonyloxygruppe, insbesondere Benzolsulfonyloxy, Paratosyloxy oder Niederalkylsulfonyloxy, bevorzugt Mesyloxy; oder für eine Acyloxygruppe wie Trifluoracetyloxy. Q steht auch für eine Hydroxygruppe oder gemäss "Synthesis" 1979, p. 561-569, für den Rest

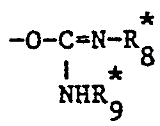 ,

worin $R^*_8$ und $R^*_9$ Organylreste, insbesondere Niederalkyl oder gegebenenfalls substituierte Phenylreste repräsentieren. M steht für Wasserstoff oder ein Metallatom, insbesondere für ein Alkalimetallatom, vorzugsweise för Natrium oder Kalium. Hal steht für Halogen, bevorzugt für Chlor oder Brom. Y bedeutet Halogen, bevorzugt Chlor oder Brom oder steht für eine Sulfat- oder Sulfonsäureestergruppe.

Das Symbol α steht stellvertretend für die Gruppierung

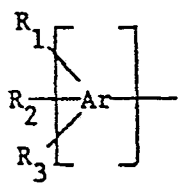

worin die Substituenten $R_1$, $R_2$, $R_3$ und Ar wie unter Formel I definiert sind.

Az repräsentiert die nachfolgende Azolylgruppe

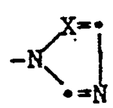

worin X für -CH= oder -N= steht.

## 1. Reaktionsschema

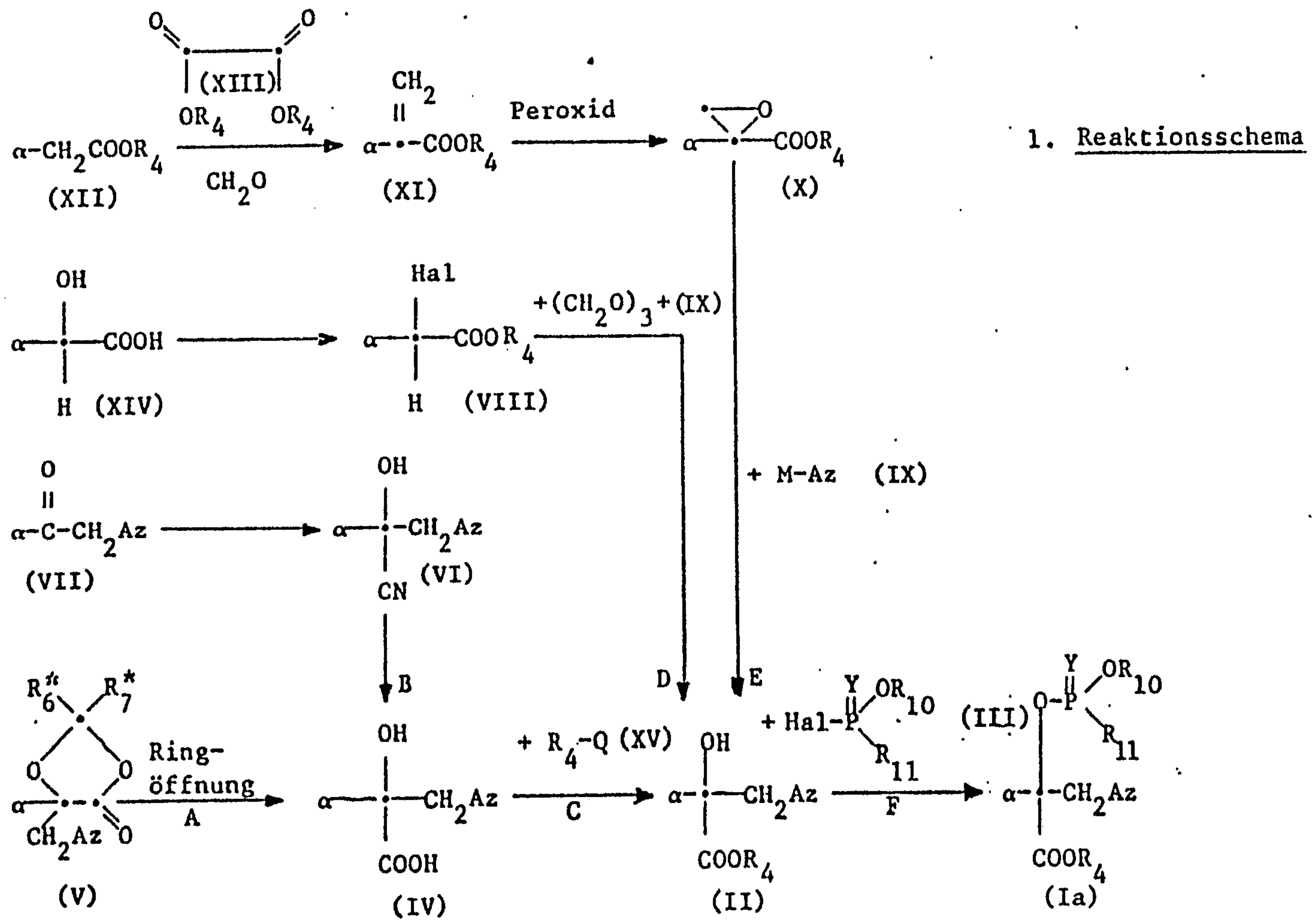

2. <u>Reaktionsschema zur Herstellung der Thioester, Amide und Hydrazide</u>

(α und Az sind wie im 1. Reaktionsschema definiert)

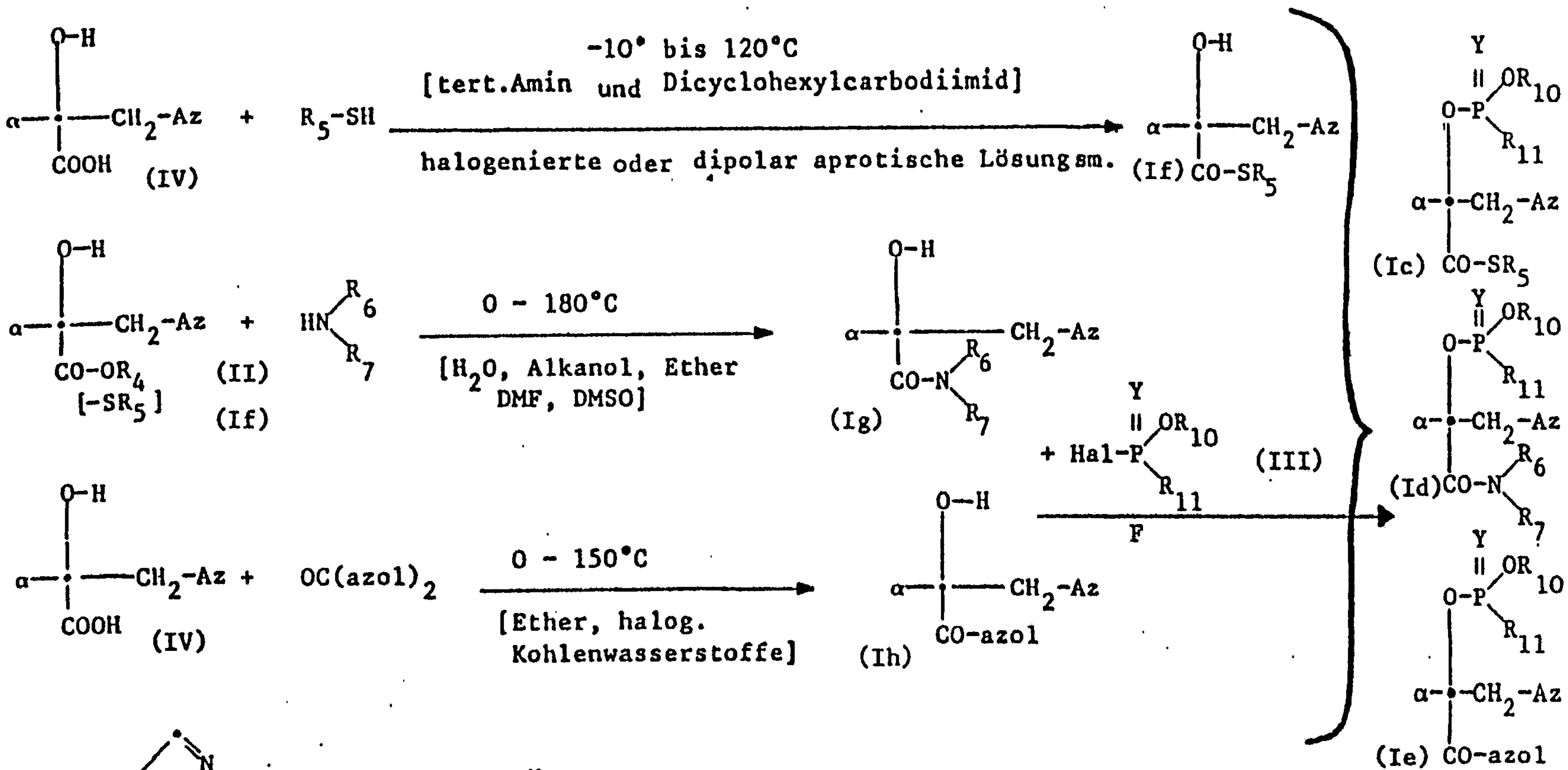


(IV) (If) (II) (If) (Ig) (IV) (Ih) (III) (Ic) (Id) (Ie)

azol= 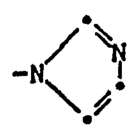 oder 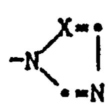

Zur Herstellung der Zwischenprodukte sowie der Endprodukte der Formel I kann man im einzelnen wie folgt vorgehen:

i) Freie α-Hydroxycarbonsäuren der Formel IV werden dadurch hergestellt, dsss man wahlweise, entweder nach Gleichung A ein Dioxolanon der Formel V oder nach Gleichung B ein Cyanhydrin der Formel VI basisch oder sauer hydrolysiert.

Die Hydrolysereaktionen A und B werden mit Säuren oder Basen vorteilhafterweise in wässrigen und/oder alkaoholischen Lösungen, also in polaren Lösungsmitteln, durchgeführt. Die Reaktionen können auch in zweiphasigen Medien durchgeführt werden. Hierbei ist die Zugabe eines üblichen Phasentransfer-Katalysators vorteilhaft. Es kommen anorganische und organische Säuren in Frage, beispielsweise Mineralsäuren wie Halogenwasserstoffsäuren, Schwefelsäure, Phosphorsäure oder Sulfonsäuren (p-Toluolsulfonsäure, Methansulfonsäure). Als Basen eignen sich organische und anorganische Basen, beispielsweise Oxide, Hydride, Hydroxide, Carbonate, Carbonsäuresalze und Alkoholate der Erdalkali- und der Alkali-Metalle, insbesondere der des Natriums und Kaliums.

Die Reaktionstemperaturen liegen bei der Ringöffnungsreaktion A im allgemeinen zwischen 0° und +140°C, bevorzugt zwischen +30° und +80°C und bei der Hydrolyse des Cyanhydrins III zwischen +60° und +140°C, bevorzugt +80° und +120°C oder jeweils am Siedepunkt des Lösungsmittels oder Lösungsmittelgemisches.

Ausgangsverbindungen der Formel V sind grösstenteils aus der EP-OS Nr. 44276 bekannt. Die noch neuen Verbindungen werden analog den dort angegebenen Methoden hergestellt.

Die Nitrile VI (Variante B) lassen sich auf übliche Art aus Arylazolylmethylketonen der Formel VII

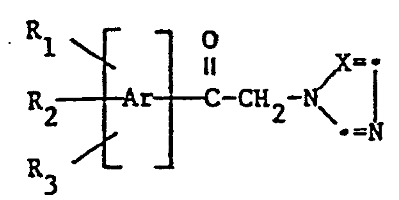

(VII)

nach Art einer Cyanohydrin-Synthese durch Reaktion mit HCN oder einem Alkalicyanid, wie z.B. KCN oder NaCN, bei 0° bis 100°C, vorteilhafterweise in Anwesenheit einer Spur einer Base (vorzugsweise $NH_4OH$ oder gasförmigem Ammoniak) oder über das korrespondierende $NaHSO_3$-Addukt VII herstellen [Org. Syntheses Coll. Vol. I, p.336, oder FR-PS 2,292,706; oder Houben-Weyl ""Methoden der organischen Chemie", Band 6/3, p. 412].

Die Nitrile VI lassen sich auch gemäss J.Org.Chem. 39, p. 914 (1974) durch Reaktion von VII mit Trimethylsilylcyanid in Gegenwart katalytischer Mengen $ZnJ_2$ und anschliessende Hydrolyse des Additionsproduktes herstellen.

Sie lassen sich auch durch Reaktion eines Ketons VII mit einem an sich bekannten Diniederalkylcyanhydrin der Formel

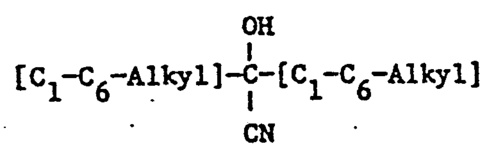

(Alkyl ist besonders Methyl, Ethyl, Propyl) bevorzugt in einem inerten Lösungsmittel oder ohne Lösungsmittel bei 50-150°C gewinnen.

Die Hydrolyse der Nitrile VI zu Säurederivaten der Formel IV kann analog zu bekannten Methoden z.B. mit konz. Chlorwasserstoffsäure erfolgen [Houben-Weyl ""Methoden der organischen Chemie, Band VIII, p. 427 ff. (1952)].

Die als Zwischenprodukte dienenden Ketone der Formel VII sind aus der DE-OS 2,431,407 bzw. der GB-PS 1,464,224 zum Teil bekannt geworden. Derartige Ketone lassen sich auch durch Hydrolyse aus entsprechenden Ketalen herstellen, z.B. aus solchen, die in einer der folgenden Publikationen genannt sind: DE-OS 2,610,022; 2,602,770; 2,930,029; 2,930,196; 2,940,133.

Noch nicht beschriebene Ketone der Formel VII lassen sich nach einer der vorstehend publizierten Methoden erhalten.

ii) Verbindungen der Formel II lassen sich gemäss Gleichung C auf übliche Art durch Veresterung des entsprechenden Säure-Derivats IV (auch in Form seines Alkalimetallsalzes) mit $R_5$-Q (XV) bei -20° bis +140°C herstellen. Für diese Reaktion sind aprotische Lösungsmittel bevorzugt. Die direkte Veresterung wird vorteilhaft mit überschüssigem Alkohol $R_5$-OH bei 0° bis 80°C in Gegenwart von Mineralsäuren oder bevorzugt von Lewis-Säuren wie Bortrifluorid-Etherat durchgeführt.

Verbindungen der Formel II lassen sich auch gemäss Gleichung D mit Paraformaldehyd bei 20° bis 140°C, vorzugsweise 40° bis 80°C, und a) mit dem gewünschten Azol der Formel IK (Imidazol oder Triazol) in Gegenwart einer Base (z.B. NaOH) oder b) mit einem Alkalisalz des Azols der Formel IX in wasserfreien Lösungsmitteln (z.B.

Dimethylsulfoxid) herstellen. Die α-Halogenessigester VIII sind durch übliche Veresterung aus den zugrundeliegenden, an sich bekannten Säuren XIV zugänglich.

Ester der Formel II lassen sich auch gemäss Gleichung E aus Oxiranen der Formel X mit einem Azol IX (M = H oder Alkalimetall) in einem inerten, bevorzugt polaren Lösungsmittel (DMF, Acetonitril, DMSO und anderen auch in Gemischen mit Kohlenwasserstoffen) bei 20° bis 100°C herstellen. Dabei können anorganische oder organische Basen zugesetzt werden [vgl. auch EP-OS 15756].

Wie im 1. Reaktionsschema skizziert, sind Oxirane der Formel X durch übliche Epoxidierung (z.B. $H_2O_2$/wss.NaOH, Peressigsäure u.a.) aus entsprechenden Alkenylverbindungen der Formel XI herstellbar. Verbindungen der Formel XI werden aus Arylessigsäureestern der Formel XII durch Reaktion mit Oxalsäureestern der Formel XIII und Formaldehyd in Gegenwart einer Base hergestellt [vg1. Helvetica Chimica Acta 30, p. 1349 (1947) und DE-OS 2,653,189].

Ester der Formel II lassen sich auch aus Säuren der Formel IV und Dimethylformamidacetal (vorzugsweise im Ueberschuss), dessen Acetalkomponente den alkoholischen Teil des Esters bilden soll, in Lösungsmitteln (z.B. ein gleichartiger wasserfreier Alkohol oder aber ein Ether) bei 0° bis 160°C herstellen [Angew. Chemie 75, p. 296 (1963) und Helv.Chim.Acta 48, 1747 (1965)].

iii) Die der Formel If entsprechenden und im 2. Reaktionsschema genannten Thioester lassen sich aus den Säuren IV mit Thioalkoholen in Gegenwart schwacher Basen (tert.Amine) in aprotischen Lösungsmitteln wie $CHCl_3$, DMF, Dichlormethan, DMSO u.a. bei -10° bis +120°C, bevorzugt 0° bis 40°C, herstellen. Diese Verbindungen werden dann mit Phosphorsäurehalogeniden der Formel 111 zu den Verbindungen der Formel Ic umgesetzt. Aus Estern (bzw. Thioestern) der Formel 11, If sind mit überschüssigem Amin $R_6$-NH-$R_7$ entsprechende Mandelsäure-amide und -hydrazide Ig herstellbar, die dann mit Verbindungen der Pormel 111 zu Verbindungen der Pormel Id reagieren. Sofern $R_6$ und $R_7$ zu einem 5- oder 6-gliedrigen Ring geschlossen sind, was für Verbindungen der Formel Ie zutrifft, führt man solch einen Heterocyclus vorteilhaft durch Reaktion der Säure IV mit 1,1'-Carbonyldi-azol bei 0° bis 150°C ein, vorzugsweise in Lösungsmitteln wie Ethern oder halogenierten Kohlenwasserstoffen. Die so erhaltenen Verbindungen der Formel Ih werden dann mit Phosphorsäurehalogeniden der Formel III zu Verbindungen der Formel Ie umgesetzt.

iv) Die freie Hydroxylgruppe in den Verbindungen IV und II ist wie vorstehend beschrieben der Phosphorylierung mit Phosphorsäurehalogeniden der Formel III zugänglich.

Sämtliche vorstehen unter i), ii), iii) und iv) geschilderten Herstellungsvarianten sind Teil vorliegender Erfindung.

Die übrigen Ausgangsprodukte der Formeln III, IX, XII, XIII und XV sind bekannt oder werden nach an sich bekannten Methoden hergestellt.

Die Verbindungen der Formel I besitzen nachbarständig zur aromatischen Gruppe Ar und zu R ein Asymmetriezentrum (*)

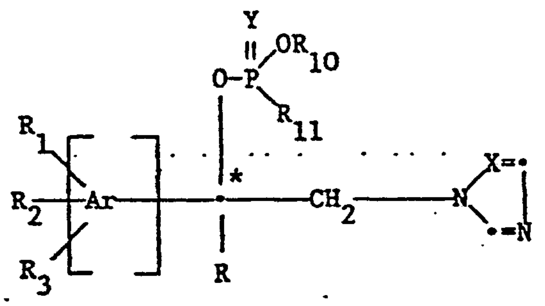

(I)

und können daher in zwei enantiomeren Formen vorliegen. Im allgemeinen entstehtbeider Herstellung dieser Substanzen ein Gemisch beider Enantiomeren, dieses lässt sichaufübliche Weise indie optischen Antipoden aufspälten. Optisch reine Antipoden erhält man beispielsweise nach Verfahrensvariante C,indem man eine optisch reine α-Hydroxycarbonsäure der Formel IV indie optisch reine Verbindung der Formel II überführt und letztere wie unter Variante F zu Verbindungen der Formel Ia phosphoryliert und in Polgereaktionen in weitere Produkte der Formel I umgewandelt. Die optisch reinen α-Hydroxysäuren IV sind z.B. über die Reaktion der racemischen Säuren IV mit optisch reinen Basen und fraktionierte Kristallisation zugänglich.

Sofern nicht speziell genannt, liegt bei der Nennung einer Verbindung der Formel I stets ein Gemisch beider enantiomeren Formen vor. Beide Antipoden zeigen unterschiedliche mikrobizide Aktivität.

Es wurde überraschend festgestellt, dass Verbindungen der Formel 1 ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen

Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Pellicularia, Puccinia); Fungi imperfecti (z.B. Botrytis, Helminthosporium, Fusarium, Septoria, Cercospora, Piricularia und Alternaria) und gegen Phycomyceten wie Pythium. Verbindungen der Formel I wirken auch gegen phytopathogene Bakterien, wie insbesondere gegen die zur Familie der Pseudomonadacese gehörenden Xanthomonas Species. Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus. Auffallend ist insbesondere eine ausgeprägte Rostwirkung bei Getreide- und Reissorten.

Die Verbindungen der Formel I eignen sich auch zur Bekämpfung von Schädlingen an Tieren, Pflanzen und im Boden.

So eignen sich die Wirkstoffe der Formel I ferner zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von phytopsthogenen Milben und Zecken der Ordnung Akarina. Verbindungen der Formel I bekämpfen pflanzenschädigende Insekten, insbesondere pflanzenschädigende Frassinsekten, in Zier- und Nutzpflanzen, insbesondere in Baumwollkulturen (z.B. Spodoptera littoralis und Heliothis virescens) und Gemüsekulturen (z.B. Leptinotarsa decemlineata und Myzus persicae).

In diesem Zusammenhang ist hervorzuheben, dass die genannten Verbindungen sich sowohl durch eine stark ausgeprägte systemische als auch Kontakt-Wirkung gegen saugende Insekten und vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Wirkstoffe der Formel I wirken darüberhinaus auch gegen Fliegen, wie z.B. Musca domestica und Mückenlarven. Ferner zeichnen sie sich durch eine breite ovizide und ovolarvizide Wirkung aus und besitzen eine wertvolle Wirkung gegen ektoparasitäre Milben und Zecken z.B. der Familien Ixodidae, Argasidae und Dermanyssidae.

Die Erfindung betrifft somit auch schädlingsbekämpfende Mittel sowie die Verwendung der Verbindungen der Formel I zur Bekämpfung von Agrar-Schädlingen, insbesondere pflanzenschädigender Pilze und Insekten bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen diser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst; (Aepfel, Birnen, Pflaumen, Pfirsiche, Mandeln, Kirschen, Erd-, Himund Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Salat, Spargel, Kohlarten, Möhren, Zwiebeln, Tomaten, Kartoffeln, Paprika; Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen Spritzpulvern löslichen Pulvern, Stäubemitteln, Granulaten durch Verkapselungen in z.B. polymeren Stoffen in bekannter Weise verarbeitet. Die Anwendungsverfahren wie Versprühen, Vernebeln, Verstäuben, Verstreuen Bestreichen oder Giessen werden gleich wie die Art der Mittel den angestrebten Zielen und den gegebenen Verhältnissen entsprechend gewählt. Günstige Aufwandmengen liegen im allgemeincn bei 50 g bis 5 kg Aktivsubstanz (AS) je ha; bevorzugt 100 g bis 2 kg AS/ha, insbesondere bei 200 g bis 600 g AS/ha.

Die Applikation solcher Mittel kann direkt auf die Pflanze oder Pflanzenteile erfolgen (Blattapplikation) oder

auf den Standort der Pflanze (Bodenapplikation) oder auf die Vermehrungsstelle, z.B. durch Saatgut-Applikation.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden). Solche Mittel sind gleichfalls Gegenstand vorliegender Erfindung.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethyl-ether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl; oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht sorptive Träger z.B. Calcit oder Dolomit in Frage. Es können auch zerkleinerte Pflanzenrückstände verwendet werden. Besonders vorteilhaft können auch Phospholipide eingesetzt werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:

"Mc Cutcheon's Detergents and Emulsifiers Annual" BC Publishing Corp., Ridgewood New Jersey, 1981.

Helmut Stache "Tensid-Taschenbuch" Carl Hanser-Verlag München/Wien 1981.

Die agrochemischen Zubereitungen enthalten in der Regel 0,1 bis 99%, insbesondere 0,1 bis 95% Wirkstoff der Formel 1, 99,9 bis 1%, insbesondere 99,8 bis 5% fester oder flüssiger Zusatzstoffe, darunter 0 bis 25%, insbesondere 0,1 bis 25% eines oder mehrerer Tenside.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittelm Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken. Temperaturen sind in Celsiusgradcn angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs. = absolut wasserfrei; DMSO = Dimethylsulfoxid; DMF = Dimethylformamid.

**Herstellungsbeispiele:**

**Beispiel H1:** Herstellung von

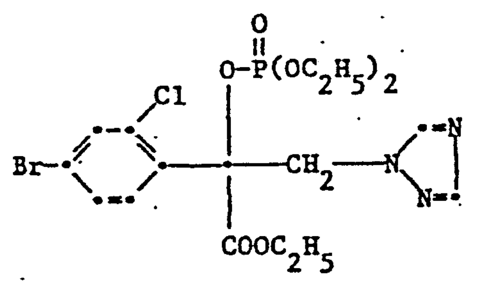

(Verb. Nr.1.3)

**2-(1H-1,2,4-Triazolylmethyl-1'-yl)-2-(2-chlor-4-bromphenyl)-2-ethoxy-carbonyl-2-phosphorsäurediethylest**

Zu einer Suspension von 1,8 g (0,04 Mol) 55%iger Natriumhydriddispersion in Paraffinöl in 50 ml THF wurden 15 g (0;04 Mol) 2-(1H-1,2,4-Triazolylmethyl-1'-yl)-2-hydroxy-2-chlor-4-bromphenylessigsäuredi-ethylester gelöst in 70 ml THF bei RT zugetropft. Nach ca. 2h war die Wasserstoffentwicklung beendet, und es wurden 8,3 g (0,048 Mol) Diethylchlorphosphat, gelöst in 70 ml THF bei RT zugetropft und das Reaktionsgemisch bei der gleichen Temperatur über Nacht gerührt. Anschliessend wurde das Gemisch 16h bei einer Badtemperatur von 80°C weitergerührt, nach dem Abkühlen auf RT wurde das Lösungsmittel im Wasserstrahlvakuum entfernt und der Rückstand in Essigsäureethylester aufgenommen und mit Wasser gewaschen. Die Esterlösung wurde über Natriumsulfat getrocknet, filtriert und in Wasserstrahlvakuum eingeengt. Dss Rohprodukt wurde säulenchromatographisch (Kieselgel/Diethylether mit steigendem Zusatz an Ethanol) gereinigt. Ausbeute: 4 g eines gelben Oels mit Brechungsindex $n^{36}_D = 1,5243$.

**Beispiel H2:** Herstellung von

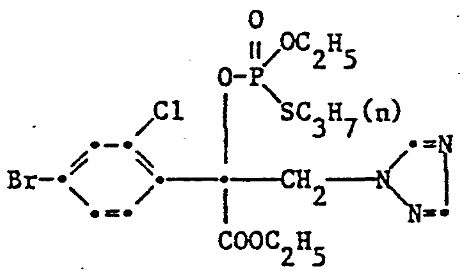

(Verb. Nr. 1.19)

**2-(1H-1,2,4-Triazolylmethyl-1′-yl)-2-(2-chlor-4-bromphenyl)-2-ethoxy-carbonyl-2-phosporsäureethyl-n-propylthioester**

Zu einer Suspension von 1,5 g (0,035 Mol) 55%iger Natriumhydriddispersion in Paraffinöl wurde bei RT eine Lösung von 13,1 g (0,035 Mol) 2-(1H-1,2,4-Triazolylmethyl-1′-yl)-2-hydroxy-2-chlor-4-bromessigsäure-diethylester in 60 ml THF zugetropft. Nach beendeter Wasserstoffentwicklung wurden 7,8 g (0,038 Mol) des entsprechenden Phosphorsäureesterchlorids, gelost in 20 ml THF, bei ca. 10-15° C zugetropft und das Reaktionsgemisch über Nacht bei RT gerührt. Nach dem Einengen im Wasserstrahlvakuum wurde der Rückstand in Essigsäureethylester gelöst, die Lösung mit Wasser gewaschen, über Natriumsulfat getrocknet und im Wasserstrahlvakuum eingeengt. Zur Abtrennung des Paraffinöls wurde der Rückstand in Acetonitril gelöst und das Paraffinöl mit Petrolether herausgelöst. Nach dem Trennen der beiden Phasen im Scheidetrichter wurde das Acetonitril im Wasserstrahlvakuum entfernt. Ausbeute: 10,5 g eines Oels mit Brechungsindex $n^{42}_D$ = 1 5359.

**Beispiel H3:** Herstellung von

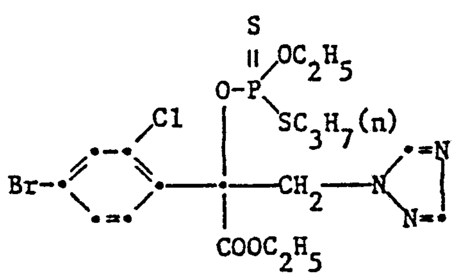

(Verb. Nr. 1.22)

**2-(1H-1,2,4-Triazolylmethyl-1′-yl)-2-(2-chlor-4-bromphenyl)-2-ethoxy-carbonyl-2-phosphorsäureethyl-n-propyldithioester**

0,9 g (0,02 Mol) 55%ige Natriumhydriddispersion wurden in 20 ml THF vorgelegt und bei RT mit 7,5 g (0,02 Mol) der entsprechenden Hydroxyverbindung, gelöst in 50 ml THF, versetzt. Nach 2h war die Wasserstoffentwicklung beendet. Nun wurden 4,8 g (0,022 Mol) des entsprechenden Chlorthiophosphorsäureesters, gelöst in 10 ml THF, bei 10°-20° C zugetropft und das Reaktionsgemisch über Nacht bei RT gerührt. Nach dem Einengen im Wasserstrahlvakuum wurde der Rückstand in Essigsäureethylester aufgenommen, mit Wasser gewaschen, über Natriumsulfat getrocknet, filtriert und im Wasserstrahlvakuum eingeengt. Der neue Rückstand wurde mit wenig Acetonitril angeschlämmt, filtriert und das Filtrat mit Petrolether nachgewaschen. Ausbeute: 6,6 g, Smp. 100°-112° C.

Nach Art der vorstehenden Beispiele und der einleitend angegebenen Herstellungsvarianten lassen sich folgende Verbindungen gemäss vorliegender Erfindung herstellen:

Tabelle 1 Verbindungen der Formel

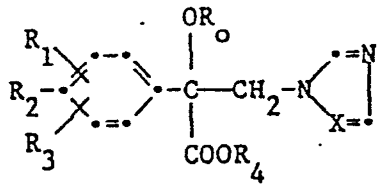

| Verb. Nr. | $R_1$, $R_2$, $R_3$ | $R_o$ | $R_4$ | X | Physikalische Daten |
|---|---|---|---|---|---|
| 1.1 | 2-Cl/4-Br | $-\overset{O}{\overset{\|}{P}}(OC_2H_5)_2$ | $-CH_3$ | N | Smp. 66-74°C |
| 1.2 | 2-Cl/4-Br | $-\overset{O}{\overset{\|}{P}}(OC_2H_5)_2$ | $-CH_3$ | CH | |
| 1.3 | 2-Cl/4-Br | $-\overset{O}{\overset{\|}{P}}(OC_2H_5)_2$ | $-C_2H_5$ | N | $n_D^{36}$: 1,5243 |
| 1.4 | 2-Cl/4-Br | $-\overset{O}{\overset{\|}{P}}(OC_2H_5)_2$ | $-C_3H_7$(n) | N | |
| 1.5 | 2-Cl/4-Br | $-\overset{O}{\overset{\|}{P}}(OC_2H_5)_2$ | $-C_4H_9$(n) | N | |
| 1.6 | 2-Cl/4-Br | $-\overset{O}{\overset{\|}{P}}(OC_2H_5)_2$ | 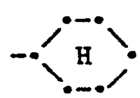 | N | |
| 1.7 | 2-Cl/4-Br | $-\overset{O}{\overset{\|}{P}}(OC_2H_5)_2$ | $-C_6H_5$ | N | |
| 1.8 | 2-Cl/4-Br | $-\overset{O}{\overset{\|}{P}}(OC_2H_5)_2$ | $-CH_2C_6H_5$ | N | |
| 1.9 | 2-Cl/4-Br | $-\overset{O}{\overset{\|}{P}}(OC_2H_5)_2$ | $-CH_2CH_2Cl$ | N | |
| 1.10 | 2-Cl/4-Br | $-\overset{O}{\overset{\|}{P}}(OC_2H_5)_2$ | $-CH_2CH_2OCH_3$ | N | |
| 1.11 | 2-Cl/4-Br | $-\overset{O}{\overset{\|}{P}}(OC_2H_5)_2$ | $-CH_2OCH_3$ | N | |

Tabelle 1 (Fortsetzung)

| Verb. Nr. | $R_1$, $R_2$, $R_3$ | $R_o$ | $R_4$ | X | Physikalische Daten |
|---|---|---|---|---|---|
| 1.12 | 2-Cl/4-Br | 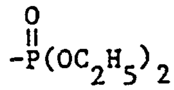 | $-CH_2SCH_3$ | N | |
| 1.13 | 2-Cl/4-Br | 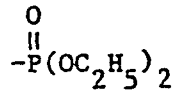 | $-CH_2CH=CH_2$ | N | |
| 1.14 | 2-Cl/4-Br | 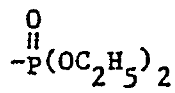 | $-CH_2COOC_2H_5$ | N | |
| 1.15 | 2-Cl/4-Br | 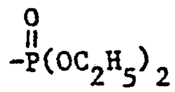 | $-CH_2COC_4H_9$(s) | N | |
| 1.16 | 2-Cl/4-Br | 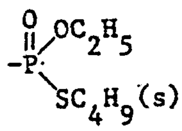 | $-C_2H_5$ | N | |
| 1.17 | 2-Cl/4-Br | 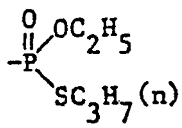 | $-CH_3$ | N | |
| 1.18 | 2-Cl/4-Br | 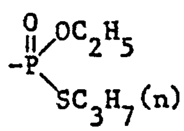 | $-C_3H_7$(i) | N | |
| 1.19 | 2-Cl/4-Br | 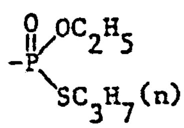 | $-C_2H_5$ | N | $n_D^{42}$: 1,5359 |
| 1.20 | 2-Cl/4-Br | 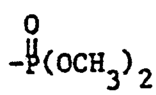 | $-C_2H_5$ | N | |
| 1.21 | 2-Cl/4-Br | 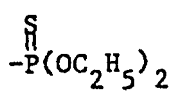 | $-C_2H_5$ | N | |
| 1.22 | 2-Cl/4-Br | 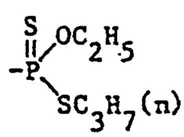 | $-C_2H_5$ | N | Smp. 100-112°C |
| 1.23 | 2-Cl/4-Br | 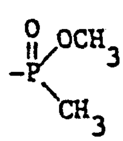 | $-C_2H_5$ | N | |

Tabelle 1 (Fortsetzung)

| Verb. Nr. | $R_1$, $R_2$, $R_3$ | $R_o$ | $R_4$ | X | Physikalische Daten |
|---|---|---|---|---|---|
| 1.24 | 2-Cl/4-Br | 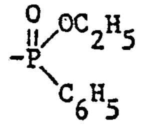 | $-C_2H_5$ | N | |
| 1.25 | 4-Cl | $-\overset{O}{\overset{\|}{P}}(OC_2H_5)_2$ | $-C_2H_5$ | N | |
| 1.26 | 3-Cl/4-Cl | $-\overset{O}{\overset{\|}{P}}(OC_2H_5)_2$ | $-C_2H_5$ | N | |
| 1.27 | 4-F | $-\overset{O}{\overset{\|}{P}}(OC_2H_5)_2$ | $-CH_3$ | N | |
| 1.28 | $2-CH_3/5-CH_3$ | $-\overset{O}{\overset{\|}{P}}(OC_2H_5)_2$ | $-CH_3$ | N | |
| 1.29 | 2-Cl/4-Cl | $-\overset{O}{\overset{\|}{P}}(OC_2H_5)_2$ | $-CH_3$ | N | Smp. 75–78°C |
| 1.30 | 2-Cl/4-Cl | $-\overset{O}{\overset{\|}{P}}(OC_2H_5)_2$ | $-CH_3$ | CH | |
| 1.31 | 2-Cl/4-Cl | $-\overset{O}{\overset{\|}{P}}(OC_2H_5)_2$ | $-C_2H_5$ | N | |
| 1.32 | 2-Cl/4-Cl | $-\overset{O}{\overset{\|}{P}}(OC_2H_5)_2$ | $-C_3H_7(n)$ | N | |
| 1.33 | 2-Cl/4-Cl | $-\overset{O}{\overset{\|}{P}}(OC_2H_5)_2$ | $-C_4H_9(n)$ | N | |
| 1.34 | 2-Cl/4-Cl | $-\overset{O}{\overset{\|}{P}}(OC_2H_5)_2$ | $-C_3H_7(i)$ | N | |
| 1.35 | 2-Cl/4-Cl | $-\overset{O}{\overset{\|}{P}}(OC_2H_5)_2$ | $-C_4H_9(t)$ | N | |
| 1.36 | 2-Cl/4-Cl | $-\overset{O}{\overset{\|}{P}}(OC_2H_5)_2$ | 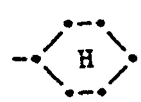 | N | |
| 1.37 | 2-Cl/4-Cl | $-\overset{O}{\overset{\|}{P}}(OC_2H_5)_2$ | $-C_6H_5$ | N | |

Tabelle 1 (Fortsetzung)

| Verb. Nr. | $R_1$, $R_2$, $R_3$ | $R_o$ | $R_4$ | X | Physikalische Daten |
|---|---|---|---|---|---|
| 1.38 | 2-Cl/4-Cl | $-\overset{O}{\overset{\|}{P}}(OC_2H_5)_2$ | $-CH_2C_6H_5$ | N | |
| 1.39 | 2-Cl/4-Cl | $-\overset{O}{\overset{\|}{P}}(OC_2H_5)_2$ | $-CH_2CH_2Cl$ | N | |
| 1.40 | 2-Cl/4-Cl | $-\overset{O}{\overset{\|}{P}}(OC_2H_5)_2$ | $-CH_2CH_2OCH_3$ | N | |
| 1.41 | 2-Cl/4-Cl | $-\overset{O}{\overset{\|}{P}}(OC_2H_5)_2$ | $-CH_2OCH_3$ | N | |
| 1.42 | 2-Cl/4-Cl | $-\overset{O}{\overset{\|}{P}}(OC_2H_5)_2$ | $-CH_2SCH_3$ | N | |
| 1.43 | 2-Cl/4-Cl | $-\overset{O}{\overset{\|}{P}}(OC_2H_5)_2$ | $-CH_2CH{=}CH_2$ | N | |
| 1.44 | 2-Cl/4-Cl | $-\overset{O}{\overset{\|}{P}}(OC_2H_5)_2$ | $-CH(CH_3)COOCH_3$ | N | |
| 1.45 | 2-Cl/4-Cl | $-\overset{O}{\overset{\|}{P}}(OC_2H_5)_2$ | $-CH_2COC_4H_9(t)$ | N | |
| 1.46 | 2-Cl/4-Cl | $-\overset{O}{\overset{\|}{P}}(OCH_3)_2$ | $-CH_3$ | N | Harz |
| 1.47 | 2-Cl/4-Cl | 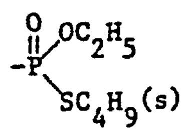 | $-CH_3$ | N | |
| 1.48 | 2-Cl/4-Cl | 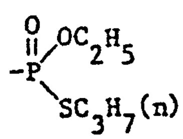 | $-C_2H_5$ | N | |
| 1.49 | 2-Cl/4-Cl | 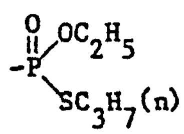 | $-C_3H_7(n)$ | N | |

Tabelle 1 (Fortsetzung)

| Verb. Nr. | $R_1$, $R_2$, $R_3$ | $R_o$ | $R_4$ | X | Physikalische Daten |
|---|---|---|---|---|---|
| 1.50 | 2-Cl/4-Cl | 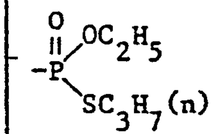 | $-CH_2CH=CH_2$ | N | |
| 1.51 | 2-Cl/4-Cl | 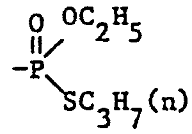 | $-CH_3$ | N | Harz |
| 1.52 | 2-Cl/4-Cl | 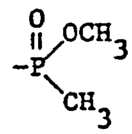 | $-CH_3$ | N | |
| 1.53 | 2-Cl/4-Cl | 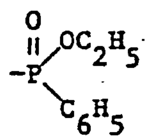 | $-CH_3$ | N | |
| 1.54 | 2-Cl/4-Cl | 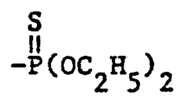 | $-CH_3$ | N | |
| 1.55 | 2-Cl/4-Cl | 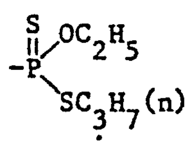 | $-CH_3$ | N | Harz |
| 1.56 | 2-Cl/4-Cl | 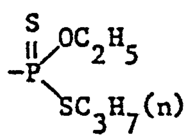 | $-C_2H_5$ | N | |
| 1.57 | 2-Cl/4-F | 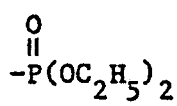 | $-CH_3$ | N | |
| 1.58 | 2-Cl/4-F | 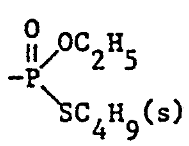 | $-CH_3$ | N | |
| 1.59 | 2-Cl/4-F | 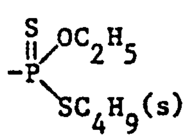 | $-CH_3$ | N | |

Tabelle 1 (Fortsetzung)

| Verb. Nr. | $R_1$, $R_2$, $R_3$ | $R_o$ | $R_4$ | X | Physikalische Daten |
|---|---|---|---|---|---|
| 1.60 | 2-Cl/4-F | 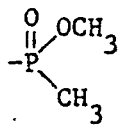 | $-CH_3$ | N | |
| 1.61 | 2-Cl/4-F | 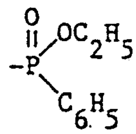 | $-CH_3$ | N | |
| 1.62 | 2-Cl/4-F | 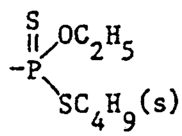 | $-CH_3$ | N | |
| 1.63 | 2-Cl/4-F | 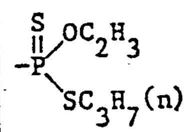 | $-CH_3$ | N | |
| 1.64 | 2-Cl/4-F | 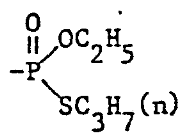 | $-CH_3$ | N | |
| 1.65 | 2-Br/4-Br | 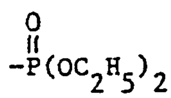 | $-C_2H_5$ | N | Harz |
| 1.66 | 2-Br/4-Br | 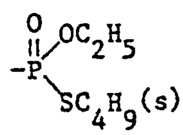 | $-C_2H_5$ | N | |
| 1.67 | 2-Br/4-Br | 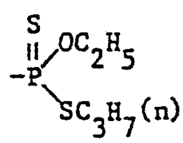 | $-C_2H_5$ | N | |
| 1.68 | 2-Br/4-Br | 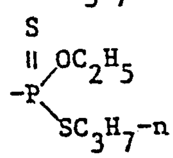 | $-C_3H_7$-i | N | Smp.113-115°C |

Tabelle 2 Verbindungen der Formel

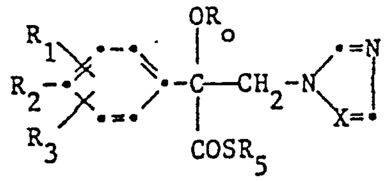

| Verb. Nr. | $R_1$, $R_2$, $R_3$ | $R_o$ | $R_5$ | X | Physikalische Daten |
|---|---|---|---|---|---|
| 2.1 | 2-Cl/4-Cl | $-\overset{O}{\overset{\|}{P}}(OC_2H_5)$ | $-CH_3$ | N | Harz |
| 2.2 | 2-Cl/4-Cl | $-\overset{O}{\overset{\|}{P}}(OC_2H_5)$ | $-C_2H_5$ | N | |
| 2.3 | 2-Cl/4-Cl | $-\overset{O}{\overset{\|}{P}}(OC_2H_5)$ | $-C_2H_5$ | CH | |
| 2.4 | 2-Cl/4-Cl | $-\overset{O}{\overset{\|}{P}}(OC_2H_5)$ | $-C_6H_5$ | N | |
| 2.5 | 2-Cl/4-Cl | $-\overset{O}{\overset{\|}{P}}(OC_2H_5)$ | $-CH_2-C_6H_5$ | N | |
| ~~2.6~~ | ~~2-Cl/4-Cl~~ | ~~$-\overset{O}{\overset{\|}{P}}(OC_2H_5)$~~ | ~~$-CH_2CH=CH_2$~~ | ~~N~~ | |
| 2.~~7~~6 | 2-Cl/4-Cl | $-\overset{O}{\overset{\|}{P}}(OC_2H_5)$ | $-C_4H_9(n)$ | N | |
| 2.~~8~~7 | 2-Cl/4-Cl | $-\overset{O}{\overset{\|}{P}}(OC_2H_5)(SC_3H_7(n))$ | $-C_2H_5$ | N | |
| ~~2.9~~ | ~~2-Cl/4-Cl~~ | ~~$-\overset{O}{\overset{\|}{P}}(OC_2H_5)(SC_3H_7(n))$~~ | ~~$-CH_2CH=CH_2$~~ | ~~N~~ | |
| 2.~~10~~8 | 2-Cl/4-Cl | $-\overset{S}{\overset{\|}{P}}(OC_2H_5)_2$ | $-C_2H_5$ | N | |
| 2.~~11~~9 | 2-Cl/4-Cl | $-\overset{S}{\overset{\|}{P}}(OC_2H_5)(SC_3H_7(n))$ | $-C_2H_5$ | N | |

Tabelle 2 (Fortsetzung)

| Verb. Nr. | $R_1$, $R_2$, $R_3$ | $R_o$ | $R_5$ | X | Physikalische Daten |
|---|---|---|---|---|---|
| 2.12 | 2-Cl/4-Cl | 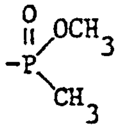 | $-C_2H_5$ | N | |
| 2.13 | 2-Cl/4-Cl | 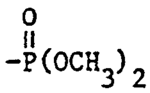 | $-C_2H_5$ | N | |
| 2.14 | 2-Cl/4-Br | 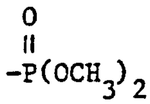 | $-C_2H_5$ | N | |
| 2.15 | 2-Cl/4-Br | 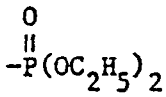 | $-C_2H_5$ | N | |
| 2.16 | 2-Cl/4-Br | 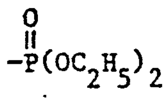 | $-C_3H_7(n)$ | N | |
| 2.17 | 2-Cl/4-Br | 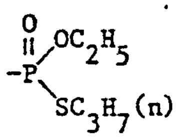 | $-C_2H_5$ | N | |
| 2.18 | 2-Cl/4-Br | 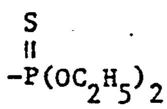 | $-C_2H_5$ | N | Harz |
| 2.19 | 2-Cl/4-Br | 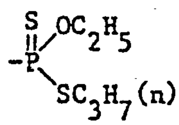 | $-C_2H_5$ | N | |
| 2.20 | 2-Cl/4-Br | 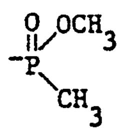 | $-C_2H_5$ | N | |
| 2.21 | 2-Cl/4-F | 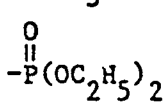 | $-C_2H_5$ | N | |
| 2.22 | 2-Cl/4-F | 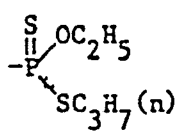 | $-C_2H_5$ | N | |
| 2.23 | 2-Cl/4-F | 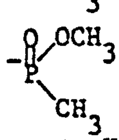 | $-C_2H_5$ | N | |
| 2.24 | 2-Cl/4-F | 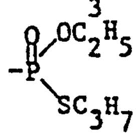 | $-C_2H_5$ | N | |

Tabelle 3 Verbindungen der Formel

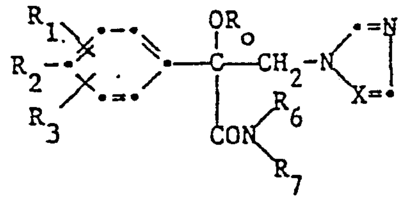

| Verb. Nr. | $R_1$, $R_2$, $R_3$ | $R_o$ | 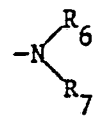 | X | Physikalische Daten |
|---|---|---|---|---|---|
| 3.1 | 2-Cl/4-Cl | $-\overset{O}{\overset{\|}{P}}(OC_2H_5)_2$ | $-NHCH_3$ | N | |
| 3.2 | 2-Cl/4-Cl | $-\overset{O}{\overset{\|}{P}}(OC_2H_5)_2$ | $-NHCH_3$ | CH | |
| 3.3 | 2-Cl/4-Cl | $-\overset{O}{\overset{\|}{P}}(OC_2H_5)_2$ | $-NHC_2H_5$ | N | |
| 3.4 | 2-Cl/4-Cl | $-\overset{O}{\overset{\|}{P}}(OC_2H_5)_2$ | $-NHCH_2C_6H_5$ | N | |
| 3.5 | 2-Cl/4-Cl | $-\overset{O}{\overset{\|}{P}}(OC_2H_5)_2$ | $-N(C_2H_5)_2$ | N | |
| 3.6 | 2-Cl/4-Cl | $-\overset{O}{\overset{\|}{P}}(CO_2H_5)_2$ | 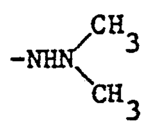 | N | |
| 3.7 | 2-Cl/4-Cl | $-\overset{O}{\overset{\|}{P}}(OC_2H_5)_2$ | $-NHNH_2$ | N | |
| 3.8 | 2-Cl/4-Cl | $-\overset{O}{\overset{\|}{P}}(OC_2H_5)_2$ | 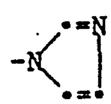 | N | |
| 3.9 | 2-Cl/4-Cl | 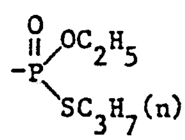 | $-NHCH_3$ | N | |
| 3.10 | 2-Cl/4-Cl | 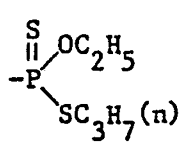 | $-NHCH_3$ | N | |
| 3.11 | 2-Cl/4-Cl | 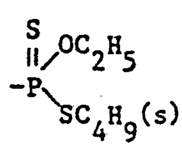 | $-NHCH_3$ | N | |

Tabelle 3 (Fortsetzung)

| Verb. Nr. | $R_1'$, $R_2$, $R_3$ | $R_o$ | 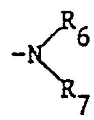 | X | Physikalische Daten |
|---|---|---|---|---|---|
| 3.12 | 2-Cl/4-Cl | 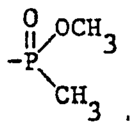 | $-NHCH_3$ | N | |
| 3.13 | 2-Cl/4-Cl | 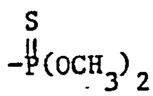 | $-NHCH_3$ | N | |
| 3.14 | 2-Cl/4-Br | 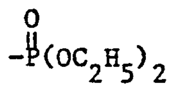 | $-NHCH_3$ | N | |
| 3.15 | 2-Cl/4-Br | 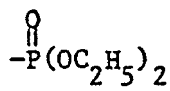 | $-NHN(CH_3)_2$ | N | |
| 3.16 | 2-Cl/4-Br | 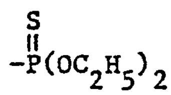 | $-NHCH_3$ | N | |
| 3.17 | 2-Cl/4-Br | 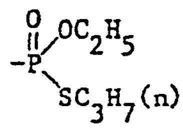 | $-NHCH_3$ | N | |
| 3.18 | 2-Cl/4-Br | 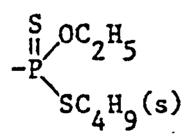 | $-NHCH_3$ | N | |
| 3.19 | 2-Cl/4-F | 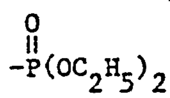 | $-NHCH_3$ | N | |
| 3.20 | 2-Cl/4-F | 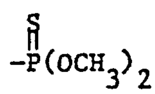 | $-NHCH_3$ | N | |
| 3.21 | 2-Cl/4-F | 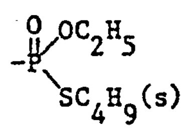 | $-NHCH_3$ | N | |
| 3.22 | 2-Cl/4-F | 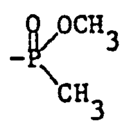 | $-NHCH_3$ | N | |
| 3.23 | 2-Br/4-Br | 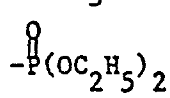 | $-NHCH_3$ | N | |
| 3.24 | 2-Br/4-Br | 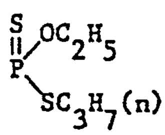 | $-NHCH_3$ | N | |

Tabelle 4 Verbindungen der Formel

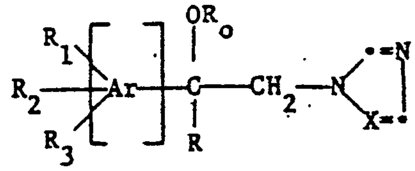

| Verb. Nr. | Ar | $R_1$, $R_2$, $R_3$ | $R_o$ | R | X | Phys.Daten |
|---|---|---|---|---|---|---|
| 4.1 | 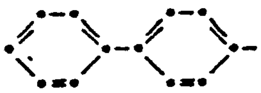 | H | 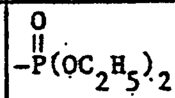 | $-COOCH_3$ | N | |
| 4.2 | 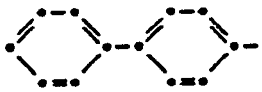 | H | 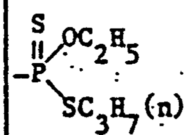 | $-COOCH_3$ | N | |
| 4.3 | 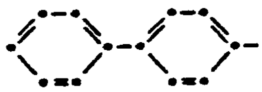 | 4-Cl | 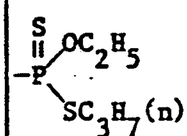 | $-COOCH_3$ | N | |
| 4.4 | 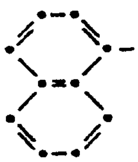 | H | 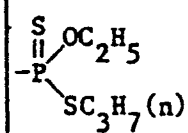 | $-COOCH_3$ | N | |
| 4.5 | 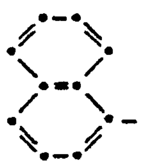 | H | 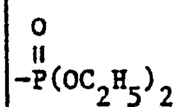 | $-COOCH_3$ | N | |
| 4.6 | 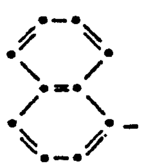 | $2-CH_3$ | 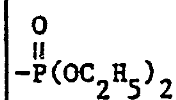 | $-COOCH_3$ | N | |
| 4.7 | 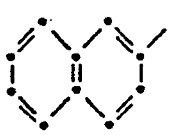 | H | 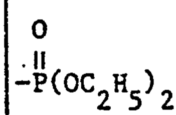 | $-COOCH_3$ | N | |
| 4.8 | 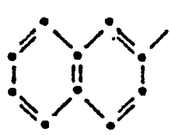 | H | 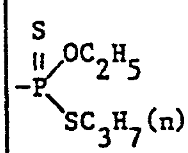 | $-COOCH_3$ | N | |

**Formulierungsbeispiele für Wirkstoffe der Formel I (%= Gewichtsprozent)**

| A. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusölpolyethylenglykolether (36 Mol Ethylenoxid) | 5% | - | - |
| Tributylphenolpolyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| B. Lösungen | a) | b) | c) | d) |
|---|---|---|---|---|
| Wirkstoff aus den Tabellen | 80% | 10% | 5% | 95% |
| Ethylenglykolmonomethylether | 20% | - | - | - |
| Polyethylenglykol MG 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| C. Granulate | a) | b) |
|---|---|---|
| Wirkstoff aus den Tabellen | 5% | 10% |
| Kaolin | 94% | - |
| Hochdisperse Kieselsäure | 1% | - |
| Atapulgit | - | 90% |

Der Wirkstoff wird in Methylenchlorid gelöst, auf den Träger aufgesprüht und das Lösungsmittel anschliessend im Vakuum abgedampft.

| D. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

| E. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

**Biologische Beispiele:**

**I. Wirkung gegen phytopathogene Pilze:**

**Beispiel B1: Wirkung gegen Puccinia graminis auf Weizen**

**a) Residual-protektive Wirkung**

Weizenpflanzen wurden 6 Tage nach der Aussaat mit einer aus Spritzpulver des Wirkstoffes hergestellten Spritzbrühe (0,02% Aktivsubstanz) besprüht. Nach 24 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

**b) Systemische Wirkung**

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Unbehandelte, aber infizierte Kontrollpflanzen zeigten einen Befall von 100%, Pflanzen, die mit Mitteln enthaltend Verbindungen der Formel I behandelt waren, zeigten nur geringen (<207.) oder keinen Befall. Die Verbindungen Nr. 1.1, 1.3, 1.19, 1.22, 1.65 unterdrückten den Pilzbefall vollständig (0-5%). Verbindung Nr = 1.3 verhinderte den Pilzbefall auch noch bei einer Konzentration von 0,002% vollständig.

**Beispiel B2: Wirkung gegen Cercospora arachidicola auf Erdnusspflanzen**

**a) Residual-protektive Wirkung**

10 - 15 cm hohe Erdnusspflanzen wurden mit einer aus Spritzpulver der Wirksubstanz hergestellten Spritzbrühe (0,002 Aktivsubstanz besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

**b) Systemische Wirkung**

Zu 10 - 15 cm hohen Erdnusspflanzen wurde eine aus Spritzpulver des Wirkstoffes hergestellte Spritzbrühe gegossen (0,06% Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 bis 4 behandelt wurden, einen stark reduzierten Cercospora-Befall. So verhinderten Verbindungen Nr. 1.1, 1.3, 1.19, 1.22, 1.65 in obigen Versuchen das Auftreten von Flecken fast vollständig (0-10%).

**Beispiel B3: Wirkung gegen Erysiphe graminis auf Gerste**

**a) Residual-protektive Wirkung**

Ca. 8 cm hohe Gerstenpflanzen wurden mit einer aus Spritzpulver des Wirkstoffes hergestellten Spritzbrühe (0,02% Aktivsubstsnz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

**b) Systemische Wirkung**

Zu ca. g cm hohen Geretenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I bzw. Verbindungen aus den Tabellen 1 bis 4 reduzierten den Pilzbefall unter 20%, während unbehandelte aber infizierte Kontrollpflanzen zu 100% befallen waren. Eine vollständige Hemmung des Pilzbefalls (0:5%) wurde mit den Verbindungen Nr. 1.1, 1.3, 1.19, 1.22 und 1.65 erzielt.

**Beispiel B4: Residual-protektive Wirkung gegen Venturia inaequalis auf Apfeltrieben**

Apfelstecklinge mit 10-20 cm langen Frischtrieben wurden mit einer aus Spritzpulver des Wirkstoffes hergestellten Spritzbrühe (0,02% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen 1.3, 1.19; 1.22 hemmten den Krankheitsbefall auf weniger als 10% und zeigten keinen Krankheitsbefall (z.B. Verb. 1.3).

Triebe an Apfelbäumen werden im Freiland in gleichem Masse geschützt, ohne in ihrer Weiterentwicklung gehemmt zu werden.

**Beispiel B5: Wirkung gegen Botrytis cinerae auf Bohnen**

**Residual-protektive Wirkung**

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100% relativer Luftfeuchtigkeit und 21°C erfolgte die Beurteilung des Pilzbefalls. Die Verbindungen aus den Tabellen 1 bis 4 hemmten in vielen Fällen die Pilzinfektion sehr stark. Bei einer Konzentration von 0,02% erwiesen sich z.B. die Verbindungen Nr. 1.1, 1.3, 1.19, 1.22 und 1.65 als voll wirksam (Krankheitsbefall 0 bis 5%).

**Beispiel B6: Wirkung gegen Piricularia oryzae auf Reis**

**Residual-protektive Wirkung**

Reispflanzen wurden nach zweiwöchiger Anzucht mit einer aus Spritzpulver des Wirkstoffs hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95-100% relativer Luftfeuchtigkeit und 24°C wurde der Pilzbefall beurteilt.

Gegenüber 100% Befall bei ungeschützten Pflanzen hemmten Verbindungen aus den Tabellen 1 bis 4 den Pilzbefall deutlich, so z.B. die Verbindung Nr. 1.1 Verbindung Nr. 1.51 verhinderte den Pilzbefall sogar noch bei einer Verdünnung auf 0,002% Aktivsubstanz vollständig (0 bis 3%).

**II. Wirkung gegen Insekten:**

**Beispiel B7: Wirkung gegen Aëdes aegypti**

Auf die Oberfläche von 150 ml Wasser, das sich in einem Behälter befindet, wird so viel einer 0,1%igen acetonischen Lösung des Wirkstoffes pipettiert, dass Konzentrationen von je 800 und 400 ppm erhalten werden. Nach Verdunsten des Acetons wird der Behälter mit 30 bis 40 2-tägigen Aedes-Larven beschickt. Nach 1, 2 und 5 Tagen wird die Mortalität geprüft.

Verbindungen der Formel I gemäss den vorstehenden Beispielen zeigen in diesem Test gute Wirkung gegen Aëdes aegypti, so zeichneten sich die Verbindungen Nr. 1.19 und 1.22 durch eine sehr hohe Mortalität (80-100%) aus.

**Beispiel B8: Insektizide Frassgift-Wirkung**

Ca. 25 cm hohe eingetopfte Baumwollpflanzen werden mit wässrigen Wirkstoffemulsionen besprüht, die den Wirkstoff in Konzentrationen von 400 bzw. 800 ppm enthalten.

Nach dem Antrocken des Sprühbelages werden die Baumwollpflanzen mit Spodoptera littoralis- bzw. Heliothis virescens-Larven im dritten larvalen Stadium besiedelt. Der Versuch wird bei 24°C und 60% relativer

Luftfeuchtigkeit durchgeführt. Nach 120 Stunden wird die %-Mortalität der Test-Insekten bestimmt.

Verbindungen der Formel I gemäss den vorstehenden Beispielen zeigen in diesem Test gute Wirkung, so zeigten die Verbindungen Nr. 1.19 und 1.22 eine hohe Mortalität (75-100%) und eine sehr gute Pflanzenverträgligkeit.

**Beispiel B9: Insektizide Wirkung: Aphis craccivora**

Bewurzelte Bohnenpflanzen werden in Töpfe, welche 60 ccm Erde enthalten, verpflanzt. Anschliessend werden 50 ml einer Versuchslösung enthaltend 25 ppm, 5 ppm bzw. 1 ppm der zu prüfenden Verbindung, direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 48 Stunden nach Versuchsbeginn. Pro Konzentrationsdosis Testsubstanz werden zwei Pflanzen, je eine in einem separsten Topf, verwendet. Der Versuch wird bei 25° C und 70% relativer Luftfeuchtigkeit durchgeführt.

Verbindungen aus den Tabellen 1 bis 4 zeigen eine gute Wirkung gegen Aphis crsccivora. So bewirkten die Verbindungen Nr.1.10 und 1.22 eine Abtötungsrate von 70 bis 100%.

**III. Akarizide Wirkung**

**Beispiel B10: Wirkung gegen Tetranychus urticae**

Phaseolus vulgaris Pflanzen werden 12 Stunden vor dem Test auf akarizide Wirkung mit einem infestierten Blattstück aus einer Massenzucht von Tetranychus urticae belegt. Die übergelaufenen beweglichenStadien werden aus einem Chromatographiezerstäuber mit dem emulgierten Test-Präparaten derart besprüht, dass kein Ablaufen der Spritzbrühe eintritt. Nach zwei und 7 Tagen werden Larven, Adulte und Eier unter dem Binokular auf lebende und tote Individuan ausgewertet; das Ergebnis wird in Prozenten ausgedrückt. Während der ""Haltezeit" stehen die behandelten Pflanzen in Gewächshauskabinen bei 25°C.

Verbindungen aus den Tabellen 1 bis 4 zeigen in obigem Test eine gute Akarizid-Wirkung gegen Tetranychus urticae, so erwies sich Verbindung Nr. 1.19 gegenüber Latven, Adulten und Eiern als voll wirksam.

**Patentansprüche**

1. Phosphorsäureester der Formel I

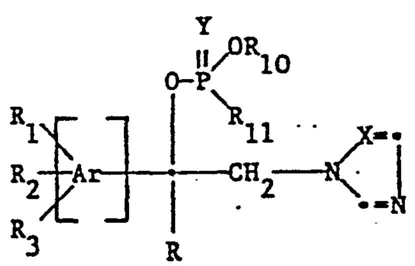

(I) ,

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen;

R eine der Gruppen $-COOR_4$, $-COSR_5$,

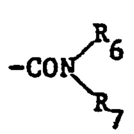

oder -CN darstellt;

$R_4$ ein unsubstituiertes oder durch Halogen substituiertes $C_2$-$C_{10}$-Alkenyl; ein unsubstituiertes oder durch Halogen substituiertes $C_2$-$C_{10}$-Alkinyl; oder eine $C_3$-$C_8$-Cycloalkylgruppe oder eine unsubstituierte oder durch

Halogen, $C_1$-$C_4$-Alkyl, $C_1$-$C_4$-Alkoxy, -CN oder -$CF_3$ substituierte Phenylgruppe bedeutet; oder aber eine $C_1$-$C_{12}$-Alkylkette darstellt, die ab $C_2$-Alkyl durch Sauerstoff oder Schwefel unterbrochen und die unsubstituiert oder durch eine der folgenden Atome oder Gruppen substituiert sein kann: Halogen, Phenyl, -COOAlkyl($C_1$-$C_4$), -COAlkyl($C_1$-$C_4$), -COPhenyl, einen ungesättigten oder gesättigten 5- oder 6-gliedrigen Ring mit Sauerstoff oder Schwefel als Heteroatom, wobei jeder Phenylrest unsubstituiert oder ein- oder mehrfach durch gleiche oder verschiedene Halogenatome substituiert ist;

$R_5$ $C_1$-$C_{10}$-Alkyl oder eine unsubstituierte oder durch Halogen, $C_1$-$C_4$-Alkyl, $C_1$-$C_4$-Alkoxy, -CN oder -$CF_3$ substituierte Phenyl- oder Benzylgruppe bedeutet;

$R_6$ und $R_7$ unabhängig voneinander Wasserstoff, $C_1$-$C_6$-Alkyl $C_3$-$C_7$-Cycloalkyl oder eine Phenyl- oder Benzylgruppe darstellen, bei denen jeweils der aromatische Ring unsubstituiert oder durch Halogen, $C_1$-$C_4$-Alkyl, $C_1$-$C_4$-Alkoxy, -CN oder -$CF_3$ substituiert ist, oder wobei einer der Substituenten $R_6$ oder $R_7$ auch die Gruppe -N($R_8$)($R_9$) bedeutet oder wobei die Substituenten $R_6$ und $R_7$ zusammen einen 5- oder 6-gliedrigen gesättigten oder ungesättigten heterocyclischen Ring bilden, der noch 1 oder 2 weitere N-Atome enthalten kann.

$R_8$ und $R_9$ unabhängig voneinander Wasserstoff, $C_1$-$C_4$-Alkyl odereinen Phenylrest bedeuten, der unsubstituiert oder durch Halogen, $C_1$-$C_4$-Alkyl, -CN oder -$CF_3$ substituiert ist;

$R_{10}$ $C_1$-$C_{12}$-Alkyl bedeutet;

$R_{11}$ für -$YR_{12}$, $C_1$-$C_{12}$-Alkyl oder Phenyl steht;

$R_{12}$ $C_1$-$C_{12}$-Alkyl bedeutet; und

Y für Sauerstoff oder Schwefel steht;

wobei pflanzenverträgliche Säureadditionssalze, quartäre Azolium- und Ammoniumsalze sowie Metallsalzkomplexe der Formel I mit eingeschlossen sind.

2. Verbindungen nach Anspruch 1 der Formel I*

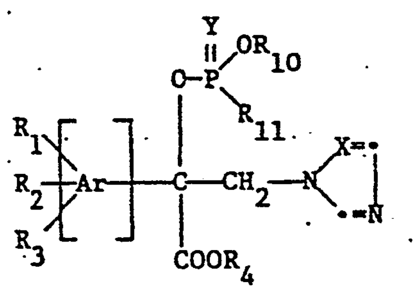

(I*) ,

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen;

$R_4$ $C_1$-$C_4$-Alkyl, Phenyl, ein durch Nitro, Halogen und/oder Methyl ein- oder mehrfach substituiertes Phenyl oder Benzyl bedeutet;

$R_{10}$ $C_1$-$C_6$-Alkyl bedeutet;

$R_{11}$ für -$YR_{12}$, $C_1$-$C_6$-Alkyl oder Phenyl steht;

$R_{12}$ $C_1$-$C_6$-Alkyl bedeutet; und

Y für Sauerstoff oder Schwefel steht;

unter Einschluss der pflanzenverträglichen Säureadditionssalze, quartäre Azolium- und Ammoniumsalze sowie Metallsalzkomplexe.

3. Verbindungen der Formel I nach Anspruch 1, worin X für das Brückenglied -CH= oder -N= steht; Ar einen Phenylgruppe bedeutet; $R_1$ in ortho-Position für Wasserstoff, Nitro, Halogen, $C_1$-$C_3$-Alkyl, $C_1$-$C_3$-Alkoxy oder $C_1$-$C_3$-Haloalkyl steht; $R_2$ in para-Position für Wasserstoff, Nitro, Halogen, $C_1$-$C_3$-Alkyl, $C_1$-$C_3$-Alkoxy oder $C_1$-$C_3$-Haloalkyl steht; $R_3$ Wasserstoff, Methyl oder Halogen bedeutet-, R eine der Gruppen

-$COOR_4$, -$COSR_5$,

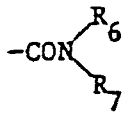

oder -CN darstellt; $R_4$ $C_1$-$C_4$-Alkyl, Phenyl, ein durch Nitro, Halogen und/oder Methyl ein- oder mehrfach substituiertes Phenyl oder Benzyl bedeutet; $R_5$ $C_1$-$C_{10}$-Alkyl oder eine unsubstituierte oder durch Halogen, $C_1$-$C_4$-Alkyl, $C_1$-$C_4$-Alkoxy, -CN oder -$CF_3$ substituierte Phenyl- oder Benzylgruppe bedeutet, $R_6$ und $R_7$ unabhängig voneinander für Wasserstoff, $C_1$-$C_3$-Alkyl, $C_3$-$C_7$-Cycloalkyl, Phenyl oder Benzyl stehen; $R_{10}$ $C_1$-$C_4$-Alkyl bedeutet; $R_{11}$ für -$YR_{12}$, $C_1$-$C_4$-Alkyl oder Phenyl steht; $R_{12}$ $C_1$-$C_4$-Alkyl bedeutet; und Y für Sauerstoff oder Schwefel steht; wobei pflanzenverträgliche Säureadditionssalze, quartäre Azolium- und Ammoniumsalze sowie Metallsalzkomplexe der Formel I mit eingeschlossen sind.

4. Verbindungen der Formel I nach Anspruch 3, worin X für das Brückenglied -N= steht;

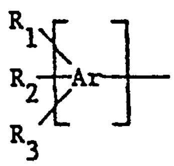

eine in ortho- und/oder para-Position durch Nitro, Fluor, Chlor, Brom, Methyl, Methoxy und/oder -$CF_3$ substituierte Phenylgruppe bedeutet; R für die Gruppe -$COOR_4$ steht; $R_4$ $C_1$-$C_6$-Alkyl, Phenyl, ein durch Nitro, Chlor, Brom, Fluor und/oder Methyl substituiertes Phenyl oder Benzyl bedeutet; $R_{10}$ $C_1$-$C_4$-Alkyl bedeutet; $R_{11}$ für -$YR_{12}$, $C_1$-$C_4$-Alkyl oder Phenyl steht-, $R_{12}$ $C_1$-$C_4$-Alkyl bedeutet; und Y für Sauerstoff oder Schwefel steht; wobei pflanzenverträglich Säureadditionssalze, quartäre Azolium- und Ammoniumsalze sowie Metallsalzkomplexe der Formel I mit eingeschlossen sind.

5. Die Verbindung

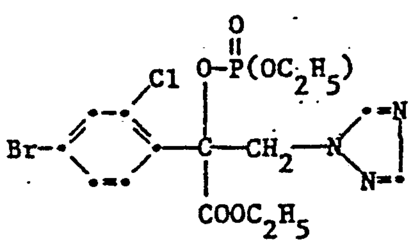

6. Die Verbindung

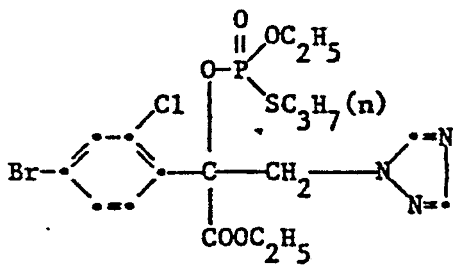

7. Die Verbindung

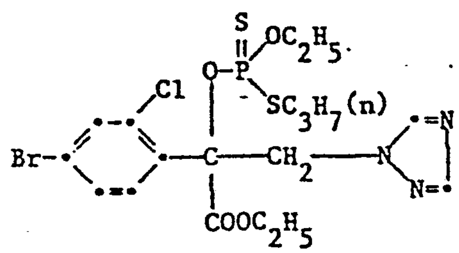

8. Die Verbindung

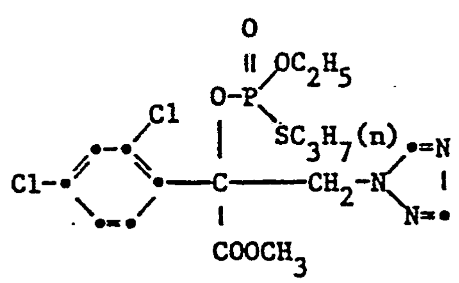

9. Die Metallsalzkomplexe der Formel I nach einem der Ansprüche 1 bis 8, mit den Metallensalzen von Kupfer, Zink, Mangan oder Zinn.

10. Verfahren zur Herstellung der in Anspruch 1 definierten Phosphorsäureester der Formel I, dadurch gekennzeichnet, dass man einen Alkohol der Formel II

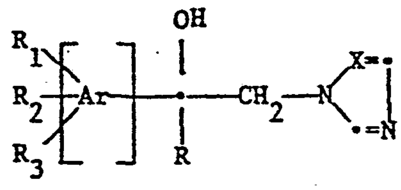

(II) ,

in An- oder Abwesenheit eines reaktionsinerten organischen Lösungs- oder Verdünnungsmittels, bei Temperaturen von −20° bis +150°C und in Gegenwart einer organischen oder anorganischen Base mit einem Phosphorsäurehalogenid der Formel III

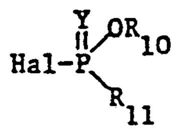

(III)

zur Reaktion bringt, wobei die Substituenten in den Formeln II und III wie unter Formel I definiert sind und Hal für ein Halogenatom steht und/oder eine verfahrensgemäss erhältliche freie Verbindung in ein pflanzenverträgliches Säureadditionssalz, quartäre Azolium- oder ein Ammoniumsalz umwandelt, ein verfahrensgemäss erhältliches pflanzenverträgliches Säureadditionssalz, quartäres Azolium- oder Ammoniumsalz in die freie Verbindung oder ein anderes pflanzenverträglich Säureadditionssalz, quartäres Azolium- oder Ammoniumsalz umwandelt, oder eine verfahrensgemäss erhältliche Verbindung bzw. ein verfahrensgemäss erhältliches Salz in einen Metallsalzkomplexe überführt.

11. Pflanzenschutzmittel zur Bekämpfung oder Verhütung eines Befalls von Pflanzen, dadurch gekennzeichnet, dass es als mindestens eine aktive Komponente eine Verbindung der Formel I gemäss einem der Ansprüche 1 bis 9 enthält, zusammen mit Trägerstoffen.

12. Verfahren zur Bekämpfung oder Verhütung eines Befalls von Kulturpflanzen durch phytopathogene Pilze, Insekten oder Akarina, dadurch gekennzeichnet, dass man eine Verbindung der Formel I gemäss einem der Ansprüche 1 bis 9 auf die Pflanze oder deren Standort appliziert.

13. Verfahren zur Herstellung eines Schädlingsbekämpfungsmittels gemäss Anspruch 11, dadurch gekennzeichnet, dass mindestens eine Verbindung der Formel I nach Anspruch 1 mit geeigneten festen und/oder flüssigen Trägerstoffen und/oder Tensiden innig vermischt wird.

**Claims:**

1. A phosphoric acid ester of the formula I

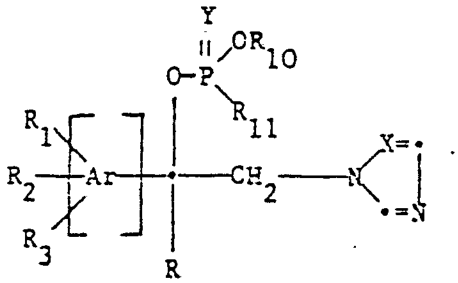

(I)

wherein
X is the bridge member -CH= or -N=,
Ar is a phenyl, diphenyl or naphthyl group,
$R_1$, $R_2$ and $R_3$ are each independently hydrogen, nitro, halogen, $C_1$-$C_3$alkyl, $C_1$-$C_3$alkoxy or $C_1$-$C_3$haloalkyl,
R is one of the groups -$COOR_4$, -$COSR_5$,

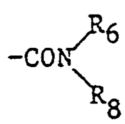

or -CN,

$R_4$ is $C_2$-$C_{10}$alkenyl which is unsubstituted or substituted by halogen; $C_2$-$C_{10}$ alkynyl which is unsubstituted or substituted by halogen. or is a $C_3$-$C_8$cycloalkyl group or a phenyl group which is unsubstituted or substituted by halogen, $C_1$-$C_4$alkyl, $C_1$-$C_4$alkoxy, -CNor -$CF_3$; or is a $C_1$-$C_{12}$alkyl chain which from $C_2$alkyl may be interrupted by oxygen or sulfur and is unsubstituted or substituted by a member selected from the group consisting of halogen, phenyl, -COO-$C_1$-$C_4$alkyl, -CO-$C_1$-$C_4$alkyl, -CO-phenyl, an unsaturated or saturated 5- or 6-membered ring containing oxygen or sulfur as heteroatom, with each phenyl moiety being unsubstituted or substituted by one or more identical or different halogen atoms,

$R_5$ is $C_1$-$C_{10}$alkyl, or is a phenyl or benzyl group, each unsubstituted or substituted by halogen, $C_1$-$C_4$alkyl, $C_1$-$C_4$alkoxy, -CN or -$CF_3$,

$R_6$ and $R_7$ are each independently hydrogen, $C_1$-$C_6$alkyl, $C_3$-$C_7$cyclo-alkyl, or a phenyl or benzyl group in each of which the aromatic ring is unsubstituted or substituted by halogen, $C_1$-$C_4$-alkyl, $C_1$-$C_4$alkoxy, -CN or -$CF_3$, or one of $R_6$ and $R_7$ is also the-N($R_8$)($R_9$) group, or both taken together form a 5- or 6-membered saturated or 2 further N-atoms,

$R_8$ and $R_9$ are each independently hydrogen, $C_1$-$C_4$alkyl or a phenyl radical which is unsubstituted or substituted by halogen, $C_1$-$C_4$-alkyl, -CN or -$CF_3$,

$R_{10}$ is $C_1$-$C_{12}$alkyl, and
$R_{11}$ is -$YR_{12}$, $C_1$-$C_{12}$alkyl or phenyl,
$R_{12}$ is $C_1$-$C_{12}$alkyl and
Y is oxygen or sulfur,
or an acid addition salt, quaternary azolium or ammonium salt or metal complex salt of formula I, which salt is tolerated by plants.

2. A compound according to claim 1, of the formula I*

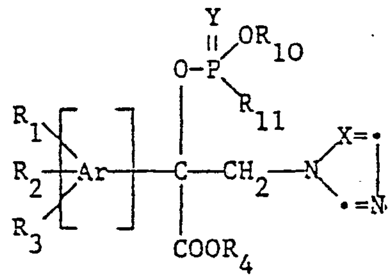

(I*)

wherein

X is the bridge member -CH= or -N=,

Ar is a phenyl, diphenyl or naphthyl group,

$R_1$, $R_2$ and $R_3$ are each independently hydrogen, nitro, halogen, $C_1$-$C_3$alkyl, $C_1$-$C_3$alkoxy or C -C haloalkyl,

$R_4$ is $C_1$-$C_4$alkyl, phenyl, or phenyl or benzyl, each substituted by one or more nitro groups, halogen atoms and/or methyl groups,

$R_{10}$ is $C_1$-$C_6$alkyl,

$R_{11}$ is -$YR_{12}$, $C_1$-$C_6$alkyl or phenyl,

$R_{12}$ is $C_1$-$C_6$alkyl, and

Y is oxygen or sulfur,

or an acid addition salt quaternary azolium or ammonium salt or metal complex salt thereof which salt is tolerated by plants.

3. A compound of the formula I according to claim 1, wherein X is the bridge member -CH= or -N=; Ar is a phenyl group; $R_1$ in the orthoposition is hydrogen, nitro, halogen, $C_1$-$C_3$alkyl, $C_1$-$C_3$alkoxy,or $C_1$-$C_3$haloalkyl; $R_2$ in the para-position is hydrogen, nitro, halogen, $C_1$-$C_3$alkyl, $C_1$-$C_3$alkoxy or $C_1$-$C_3$haloalkyl; $R_3$ is hydrogen, methyl or halogen; R is a group -$COOR_4$, -$COSR_5$,

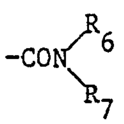 ,

or -CN; $R_4$ is $C_1$-$C_4$alkyl, phenyl, or phenyl or benzyl, each substituted by one or more nitro groups, halogen atoms and/or methyl groups; $R_5$ is $C_1$-$C_{10}$alkyl, or phenyl or benzyl, each unsubstituted or substituted by halogen, $C_1$-$C_4$alkyl, $C_1$-$C_4$alkoxy, -CN or -$CF_3$; each of $R_6$ and $R_7$ independently is hydrogen, $C_1$-$C_3$alkyl, $C_3$-$C_7$cycloalkyl, phenyl or benzyl; $R_{10}$ is $C_1$-$C_4$alkyl; $R_{11}$ is -$YR_{12}$, $C_1$-$C_4$alkyl or phenyl; $R_{12}$ is $C_1$-$C_4$alkyl; and Y is oxygen or sulfur, or an acid additionsalt, quaternary azolium or ammonium salt or metal complex salt thereof, which salt is tolerated by plants.

4. A compound of the formula I according to claim 3, wherein X is the bridge member -N=; the grouping

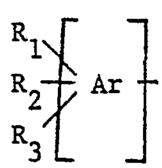

is a phenyl group which is substituted in the ortho- and/or para-position by nitro, fluorine, chlorine, bromine, methyl, methoxy and/or $CF_3$; R is the -$COOR_4$ group; $R_4$ is $C_1$-$C_4$alkyl, phenyl, or phenyl orbenzyl, each substituted by nitro, chlorine, bromine, fluorine and/or methyl; $R_{10}$ is $C_1$-$C_4$alkyl-, $R_{11}$ is -YR $_{12}$, $C_1$-$C_4$alkyl or phenyl-, $R_{12}$ is $C_1$-$C_4$-alkyl; and Y is oxygen or sulfur, or an acid addition salt, quaternary azolium or ammonium salt or metal complex salt thereof, which salt is tolerated by plants.

5. The compound

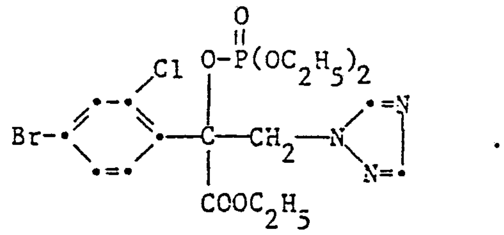

6. The compound

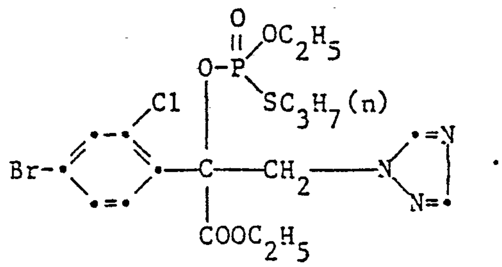

7. The compound

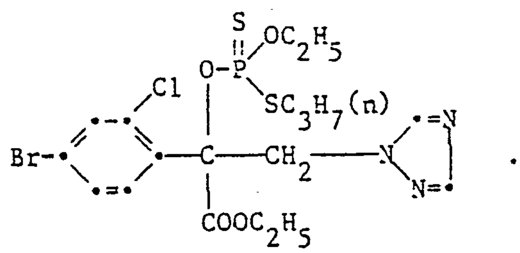

8. The compound

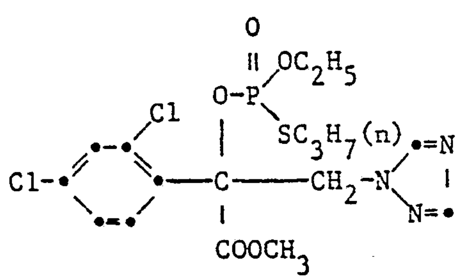

9. A metal complex salt of the formula I according to any one of claims 1 to 8 with a salt of copper, zinc, manganese or tin.

10. A process for the preparation of a phosphoric acid ester of the formula 1 as defined in claim 1, which comprises reacting an alcohol of the formula II

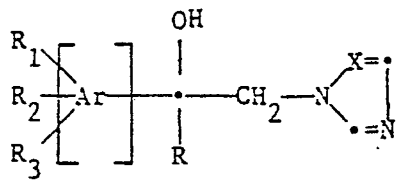

(II)

with a phosphoryl halide of the formula III

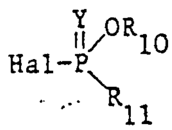

(III)

in which formulae II and III the substituents are as defined for formula I and Hal is a halogen atom, in the presence or absence of an inert organic solvent or diluent in the temperature range from -20° to +150°C and in the presence of an organic or inorganic base, and/or converting a free compound obtainable by the process into an acid addition salt or a quaternary azolium or smmonium salt, or converting an acid addition salt or a quaternary azolium or ammonium salt obtainable by the process into the free compound or into another acid addition salt,quaternary azolium or ammonium salt, or converting a free compound or a salt obtainable by the process of the invention into a metal complex salt, the salts being tolerated by plants.

11. A pesticidal composition for controlling or preventing infestation of plants by pests, which composition contains as active ingredient at least one compound of the formula I as claimed in any one of claims 1 to 9, together with suitable carriers therefor.

12. A method of controllin phytopatogenic fungi, insects or acarina or of protecting cultivated plants from attack by such pests, which comprises applying to said plants or to the locus thereof a pestcidally effective amount of a compound of the formula I as claimed in any one of claims 1 to 9.

13. A process for the preparation of a pesticidal composition as defined in claim 11, wich comprises intimately mixing at least one compound of the formula I as claimed in claim 1 with suitable solid and/or liquid carriers and/or surfactants.

**Revendications**

1.- Esters phosphoriques de formule I :

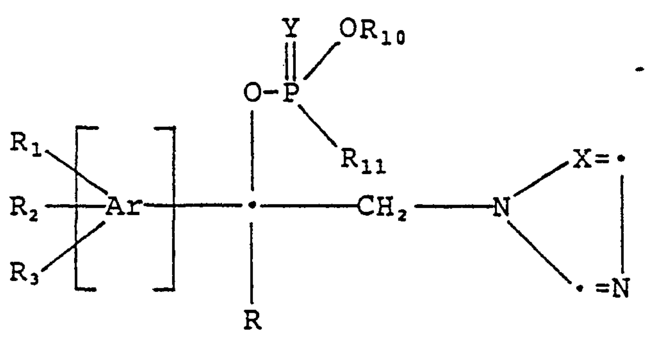

I

dans laquelle:
X représente le pont -CH= ou -N=;

Ar représente un groupe phényle, diphényle ou naphtyle;

$R_1$, $R_2$ et $R_3$ représentent chacun, indépendamment les uns des autres, l'hydrogène, un groupe nitro, un halogène, un groupe alkyle en $C_1$-$C_3$, alcoxy en $C_1$-$C_3$ ou halogénoalkyle en $C_1$-$C_3$;

R représente l'un des groupes $-COOR_4$, $-COSR_5$,

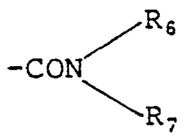

ou -CN ;

$R_4$ représente un groupe alcényle en $C_2$-$C_{10}$ non substitué ou substitué par des halogenes; un groupe alcynyle en $C_2$-$C_{10}$ non substitué ou substitué par des halogenes; ou bien un groupe cycloalkyle en $C_3$-$C_8$ ou un groupe phényle non substitué ou substitué par des halogènes, des groupes alkyle en $C_1$-$C_4$, alcoxy en $C_1$-$C_4$, -CN ou -$CF_3$; ou bien encore une chaîne alkyle en $C_1$-$C_{12}$ qui, à partir d'alkyle en $C_2$, peut être interrompue par l'oxygène ou le soufre, et qui est non substituée ou peut être substituée par l'un des atomes ou groupes suivants: halogènes, phényle, -COO-alkyle en $C_1$-$C_4$, -COalkyle en $C_1$-$C_4$, CO-phényle, un cykcle insaturé ou saturé à 5 ou 6 chaînons contenant l'oxygène ou le soufre en tant qu'hétéroatome, chaque groupe phényle étant non substitué ou substitué une ou plusieurs fois par des atomes d'halogènes identiques ou différents;

$R_5$ représente un groupe alkyle en $C_1C_{10}$ ou en groupe phényle ou benzyle non substitué par des halogènes, des groupes alkyle en $C_1$-$C_4$, alcoxy en $C_1$-$C_4$, -CN ou -$CF_3$;

$R_6$ et $R_7$ représentent chacun indépendamment l'un de l'autre, l'hydrogène, un groupe alkyle en $C_1$-$C_6$, cycloalkyle en $C_3$-$C_7$ ou un groupe phényle ou benzyle qui peut être non substitué sur le noyau aromatique ou substitué sur le noyau aromatique par des halogènes, des groupes alkyle en $C_1$-$C_4$, alcoxy en $C_1$-$C_4$, -CN ou -$CF_3$; ou bien l'un des substituants $R_6$ et $R_7$ représentent également le groupe $-N(R_8)(R_9)$ ou bien les substituants $R_6$ et $R_7$ forment ensemble un noyau hétérocyclique saturé ou insaturé à 5 ou 6 chaînons qui peut encore contenir un ou deux autres atomes d'azote;

$R_8$ et $R_9$ représentent chacun, indépendamment l'un de l'autre, l'hydrogène, un groupe alkyle en $C_1C_4$ ou un groupe phényle qui est non substitué ou substitué par des halogènes, des groupes alkyle en $C_1$-$C_4$, -CN ou -$CF_3$;

$R_{10}$ représente ung roupe alkyle en $C_1$-$C_{12}$ ou phényle;

$R_{12}$ représente un groupe alkyle en $C_1$-$C_{12}$; et

Y représente l'oxygène ou le soufre;

y compris leurs sels formés par addition avec des acides, sels d'azolium et d'ammonium quaternaires et complexes de sels métalliques de formule I, tolérés par les végétaux.

2.-Composés selon la revendication 1, répondant à la formule I*:

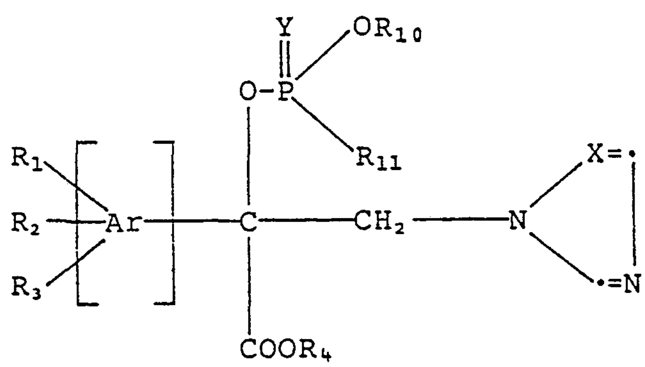

I*

dans laquelle:

X représenten le pont -CH= ou -N=;

Ar représente un groupe phényle, diphényle ou naphtyle;

$R_1$, $R_2$ et $R_3$ représentent chacun, indépendamment les uns des autres, l'hydrogène, un groupe nitro, un halogène, un groupe alkyle en $C_1$-$C_3$ ou halogénoalkyle en $C_1$-$C_3$;

$R_4$ représente un groupe alkyle en $C_1$-$C_4$, phényle, un groupe phényle ou benzyle mono- ou poly.substitué par des groupes nitro, des halogènes et/ou des groupes méthyle;

$R_{10}$ représente un groupe alkyle en $C_1$-$C_6$;

$R_{11}$ représente un groupe $-YR_{12}$, alkyle en $C_1$-$C_6$ ou phényle;

$R_{12}$ représente un groupe alkyle en $C_1$-$C_6$; et

Y représente l'oxygène ou le soufre;

y compris les sels formés par addition avec des acides, sels d'azolium et d'ammonium quaternaires et

complexes de sels métalliques, tolérés par les végétaux.

3.- Composés de formule I selon la revendication 1, dans laquelle :

X représente le pont -CH= ou -N=;

Ar représente un groupe phényle;

$R_1$ représente en position ortho, l'hydrogène, un groupe nitro, un halogène, un groupe alkyle en $C_1$-$C_3$, alcoxy en $C_1$-$C_3$ ou halogénoalkyle en $C_1$-$C_3$;

$R_2$ représente en position para, l'hydrogène, un groupe. nitro, un halogène, un groupe alkyle en $C_1$-$C_3$, alcoxy en $C_1$-$C_3$ ou halogénoalkyle en $C_1$-$C_3$;

$R_3$ représente l'hydrogène, un groupe méthyle ou un halogène;

R représente l'un des groupes $-COOR_4$, $-COSR_5$,

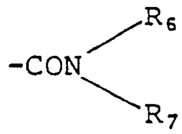 ou $-CN$ ;

$R_4$ représente un groupe alkyle en $C_1$-$C_4$, phényle, un groupe phényle ou benzyle mono-ou poly-substitué par des groupes nitro, des halogènes et/ou des groupes méthyle;

$R_5$ représente un groupe alkyle en $C_1$-$C_{10}$ ou un groupe phényle ou benzyle non substitué ou substitué par des halogènes, des groupes alkyle en $C_1$-$C_4$, alcoxy en $C_1$-$C_4$, -CN ou $-CF_3$;

$R_6$ et $R_7$ représentent chacun, indépendamment l'un de l'autre, l'hydrogène, un groupe alkyle en $C_1$-$C_3$, cycloalkyle en $C_3$-$C_7$, phényle ou benzyle;

$R_{10}$ représente un groupe alkyle en $C_1$-$C_4$;

$R_{11}$ représente un groupe $-YR_{12}$, alkyle en $C_1$-$C_4$ ou phényle;

$R_{12}$ représente un groupe alkyle en $C_1$-$C_4$; et

Y représente l'oxygène ou le soufre;

y compris les sels formés par addition avec des acides, sels d'azolium et d'ammonium quaternaires et complexes de sels métalliques de formule I, tolérés par les végétaux.

4.- Composés de formule I selon la revendication 3, dans lesquels X représente le pont -N=;

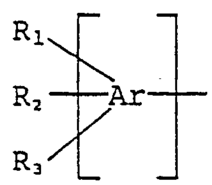

représente un groupe phényle substitué en position ortho et/ou para par des groupes nitro, le fluor, le chlore, le brome, des groupes méthyle, méthoxy et/ou $-CF_3$;

R représente le groupe $-COOR_4$;

$R_4$ représente un groupe alkyle en $C_1$-$C_6$, phényle, un groupe phényle ou benzyle substitué par des groupes nitro, le chlore, le brome, le fluor et/ou des groupes méthyle;

$R_{10}$ représente un groupe alkyle en $C_1$-$C_4$;

$R_{11}$ représente un groupe $-YR_{12}$, alkyle en $C_1$-$C_4$ ou phényle;

$R_{12}$ représente un groupe alkyle en $C_1$-$C_4$; et

Y représente l'oxygène ou le soufre;

y compris les sels formés par addition avec des acides, sels d'azolium et d'ammonium quaternaires et complexes de sels métalliques de formule I tolérés par les végétaux.

5.- Le composé

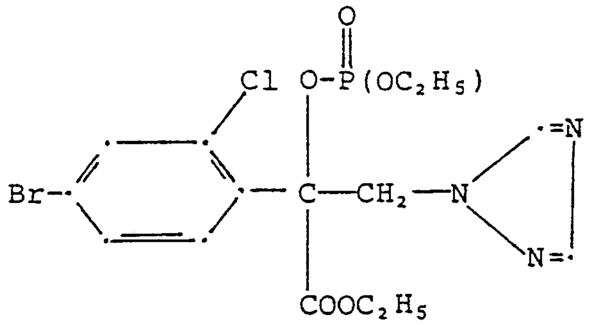

6.- Le composé

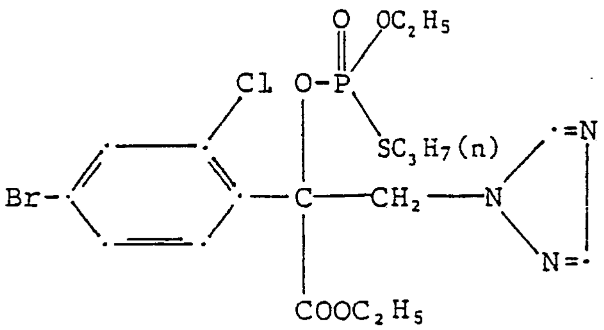

7.- Le composé

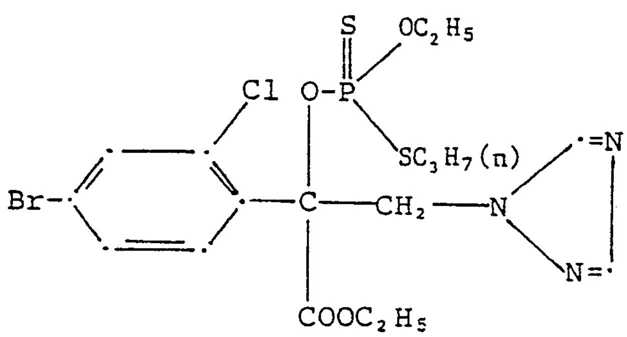

8.- Le composé

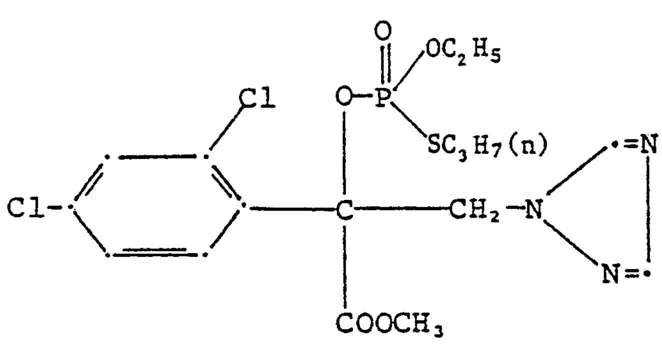

9.- Les complexes de sels métalliques de formule I selon l'une des revendications 1 à 8, des sels métalliques de cuivre, de zinc, de manganèse ou d'étain.

10.- Procédé de préparation des esters phosphoriques de formule I définis dans la revendication 1, caractérisé en ce que l'on fait réagir un alcool de formule II :

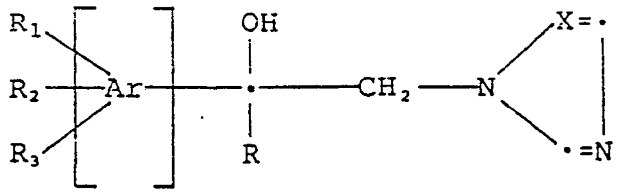

II

en présence ou en l'absence d'un solvant ou diluant organique inerte dans la réaction, à des températures de −20 à +150°C et en présence d'une base organique ou minérale, avec un halogénure d'acide phosphorique de formule III :

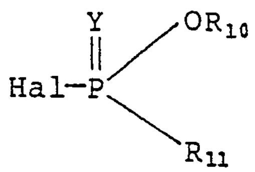

III

les substituants dans les formules II et III ayant les significations indiquées en référence à la formule I et Hal représentant un atome d'halogène, et/ou on convertit un composé libre obtenu par ce procédé en un sel formé par addition avec un acide, sel d'azolium ou sel d'ammonium quaternaire tolérés par les végétaux, on convertit un sel d'acide, sel d'azolium ou d'ammonium quaternaire tolérés par les végétaux obtenus par ce procédé, en le composé libre ou en un autre sel d'acide, sel d'azolium ou d'ammonium quaternaire tolérés par les végétaux, ou bien on convertit un composé obtenu par ce procédé ou un sel obtenu par ce procédé en un complexe de sel métallique.

11.- Agent de protection des plantes pour le traitement ou la prévention d'une infection des végétaux, caractérisé en ce qu'il contient au moins un composant actif consistant en un composé de formule I selon l'une des revendications 1 à 9, avec des véhicules.

12.- Procédé pour traiter ou prévenir une infection de plantes cultivées par des mycètes phytopathogènes, des insectes ou des acariens, caractérisé en ce que l'on applique sur la plante ou son habitat un composé de formule I selon l'une des revendications 1 à 9.

13.- Procédé de préparation d'un produit pesticide selon la revendication 11, caractérisé en ce que l'on mélange intimement au moins un composé de formule I selon la revendication 1 avec des véhicules et/ou agents tensioactifs solides et/ou liquides appropriés.